# EUROPEAN PATENT SPECIFICATION

(11) **EP 4 329 667 B1**
(45) Date of publication and mention of the grant of the patent: **06.05.2026**
(21) Application number: 22724777.2
(22) Date of filing: 25.04.2022
(51) Int. Cl.: A61L 27/38, A61L 27/54, A61L 27/56, A61M 37/00, A61F 2/02

(54) **IMPLANTABLE DEVICE AND THERAPEUTIC SYSTEM WITH PASSIVE OXYGENATION**
IMPLANTIERBARE VORRICHTUNG UND THERAPEUTISCHES SYSTEM MIT PASSIVER SAUERSTOFFANREICHERUNG
DISPOSITIF IMPLANTABLE ET SYSTÈME THÉRAPEUTIQUE À OXYGÉNATION PASSIVE

(30) Priority: 27.04.2021 US 202163180332 P; 03.03.2022 EP 22315043
(43) Date of publication of application: 06.03.2024
(73) Proprietor: Sanofi, 75017 Paris (FR)
(72) Inventor: VEDRINE, Lionel, Somerville, Massachusetts 02143 (US); LASKAR, Christina, Allendale, New Jersey 07401 (US); DHAL, Pradeep, Bridgewater, New Jersey 08807 (US); NELSON, Craig Harvey, Baldock SG7 6SU (GB); HUMPHRIES, Mark Robson, Walden Essex CB10 1PL (GB); LEE, Carys Eleri, Cambridge CB22 5DU (GB); HARDING, Nicholas Oliver, Birmingham B1 1DW (GB)
(74) Representative: Fish & Richardson P.C.
(86) International application number: PCT/EP2022/060830
(87) International publication number: WO 2022/229058

(56) References cited:
- WO-A1-2020/068852
- WO-A1-2020/243665
- US-A1- 2018 126 134
- US-A1- 2019 328 289
- US-A1- 2020 179 121

## Description

### Technical Field

This disclosure relates to implantable devices that provide a therapeutic agent to treat a disease, such as implants that provide beta cell replacement therapy.

### Background

In patients with type 1 or type 2 diabetes, either or both of mass and function of beta cells may be diminished, which can lead to insufficient insulin secretion and hyperglycemia. Cellular implants have been considered for treating such conditions. For example, a cellular implant may include a housing containing beta cells that are capable of producing insulin. Once the cellular implant is implanted into a patient's body, some or all of the cells within the housing may lack an adequate supply of oxygen. Therefore, cells within a cellular implant can be particularly vulnerable to death due to insufficient oxygen. Cell survival during this period is especially challenged in cases where a cellular implant is relatively large or has a relatively high cell density that locates some cells relatively far away from the surrounding housing and vascularization.

US 2019/328289 A1 describes an encapsulation device system for therapeutic applications such as regulating blood glucose. The system may comprise an encapsulation device with a first oxygen sensor integrated inside the device and a second oxygen sensor disposed on an outer surface of the device, wherein the sensors allow for real-time measurements (such as oxygen levels) related to cells (e.g., islet cells, stem cell derived beta cells, etc.) housed in the encapsulation device. The system may also feature an exogenous oxygen delivery system operatively connected to the encapsulation device via a channel, wherein the exogenous oxygen delivery system is adapted to deliver oxygen to the encapsulation device.

US 2018/126134 A1 describes implantable encapsulation devices for housing a biological moiety or a therapeutic device that contains a biological moiety. Particularly, aspects of the description are directed to an implantable apparatus that includes a distal end, a proximal end, a manifold including at least one access port positioned either at the distal end or the proximal end, and a plurality of containment tubes affixed to the manifold and in fluid communication with the at least one access port. Additionally, the encapsulation device may contain a flush port and a tube that are fluidly connected to the manifold. The containment tubes may contain therein a biological moiety (e.g., cells) or a therapeutic device (e.g. a cell encapsulation member).

WO 2020/243665 A1 describes a biocompatible membrane composite including a cell impermeable layer and a mitigation layer. The cell impermeable layer is impervious to vascular ingrowth and prevents cellular contact from the host. Additionally, the mitigation layer includes solid features. The mitigation layer has therein bonded solid features. The cell impermeable layer and the mitigation layer can be intimately bonded or otherwise connected to each other to form a composite layer having a tight/open structure. A reinforcing component may optionally be positioned external to or within the biocompatible membrane composite to provide support to and prevent distortion. The biocompatible membrane composite may be used in or to form a device for encapsulating biological entities, including pancreatic lineage type cells such as pancreatic progenitors.

WO 2020/068852 A1 describes an implantable medical device and methods for making and using the same. The device comprises a central hub structure in communication with at least one housing or pod capable of containing cells and therapeutic materials. Also described are membrane structures and methods of forming the same, the membranes comprising a gradient of varying porosity for use with the described devices, as well as other uses.

US 2020/179121 A1 describes an implantable medical device comprising a liquid rope coil scaffold. The implant may consist essentially of the scaffold, where the scaffold is the implant and pores in the scaffold may incorporates one or more agents (i.e. drugs, growth factors), or the scaffold may comprise only part of the medical device, for example an implant that is partly or fully covered with a layer of the scaffold. The porosity of the scaffold may be tailored to suit the application, for example a porosity that is tailored to hold and release drug or biological molecules in vivo, a porosity to provide a surface roughness that is conducive to promotion of in-vivo bio-integration (for example vascularisation) or prevention of fibrosis, or a porosity that provides structural strength. The scaffold may be essentially tubular, or may be provided as a planar structure, or may be any shape and can be used to coat, fully or partially any shape or size of medical implant.

### Summary

An implantable device and a therapeutic system according to the present invention are defined by claims 1 and 13, respectively. Further advantageous developments of the present invention are defined by the dependent claims.

In general, this disclosure relates to implantable devices that provide a therapeutic agent to treat a disease, such as implants that provide beta cell replacement therapy (BCRT). In an example embodiment, an implantable device includes a porous container that houses human beta cells capable of producing insulin to treat diabetes or hemophilia. The implantable device provides immunoisolation of the cells and promotes vascularization along an exterior surface. Furthermore, the implantable device is advantageously designed to ensure that oxygen is provided to the cells at a level that is sufficient for the cells to remain viable (e.g., to survive and to perform their therapeutic function) during a vascularization period following implantation within a patient's body. The implantable device according to the present invention is designed to provide for passive oxygenation of the cells. In a comparative example, the implantable device can provide both for passive and active oxygenation of the cells.

In a first aspect, an implantable device for providing a therapeutic agent includes a container configured to contain multiple cells capable of producing the therapeutic agent within an interior region of the container. The container defines first pores defined by an interior wall portion of the container, the first pores having a first average size that (i) allows passage of the therapeutic agent through the first pores and (ii) prevents passage of immune cells through the first pores. The container further defines second pores defined by an exterior wall portion of the container, the second pores having a second average size that is larger than the first average size, and the second pores being sized to promote vascularization along the exterior wall portion. The implantable device further includes a substance capable of reacting to generate oxygen for the multiple cells within the interior region of the container.

Embodiments may include one or more of the following features.

In some embodiments, the implantable device has an external surface area to volume ratio of about 200 to about 5,000.

In some embodiments, the container includes a tubular wall that includes the interior and exterior wall portions.

In some embodiments, the tubular wall further defines third pores arranged radially between the first and second pores, the third pores having a third average pore size that is greater than the first average pore size and less than the second average pore size.

In some embodiments, the pores defined by the tubular wall gradually increase in size from an interior surface of the tubular wall to an exterior surface of the tubular wall.

In some embodiments, the container includes one or more of polycaprolactone, PTFE, ePTFE, nylon, polyether-ketone, polyether sulfone, polyester, polyvinylidene difluoride, and polysiloxane.

In some embodiments, the interior wall portion includes a tubular member and the exterior wall portion includes a coating that surrounds the tubular member.

In some embodiments, the tubular member has a first material formulation and the coating has a second material formulation that is different from the first material formulation.

In some embodiments, the first material formulation includes one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups.

In some embodiments, the second material formulation includes one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In some embodiments, the second average size of the second pores locates vasculature along the second pores.

In some embodiments, the second average size of the second pores allows passage of the therapeutic agent through the exterior wall portion to vasculature adjacent the implantable device.

In some embodiments, the first pores have widths in a range of about 10 nm to about 400 nm.

In some embodiments, the second pores have widths in a range of about 2 µm to about 60 µm.

In some embodiments, the interior region has a width in a range of about 100 µm to about 2 mm.

In a not claimed comparative example, the interior region can be wide enough to accommodate multiple of the cells across a plane that is perpendicular to a central axis of the container.

In some embodiments, the container has a wall thickness in a range of about 1 µm to about 100 µm.

In some embodiments, the implantable device has a generally tubular shape.

In a not claimed comparative example, the implantable device includes a tube that has a linear configuration. According to the present invention, the implantable device includes a tube that has a spiral configuration or a helical configuration.

In some embodiments, ends of the tube are sealed.

In some embodiments, the tube is a first tube, and the implantable device further includes a second tube disposed inside of the first tube, wherein the first and second tubes together define an annular lumen for containing the multiple cells, and wherein multiple particles are disposed within a core region of the second tube.

In some embodiments, the exterior wall portion is coated with a growth factor that promotes vascularization.

In some embodiments, the implantable device further includes multiple particles including the substance, wherein the multiple particles are part of a coating applied to an inner surface of the interior wall portion.

In some embodiments, the exterior wall portion is coated with a growth factor that promotes vascularization.

In some embodiments, the growth factor is covalently linked to the exterior wall portion.

In some embodiments, the growth factor is electrostatically bound to the exterior wall portion.

In some embodiments, the growth factor is site-specifically bound to the exterior wall portion.

In some embodiments, the growth factor is selected from the group consisting of: vascular endothelial growth factor (VEGF), placenta derived growth factor (PDGF), transforming growth factor beta (TGFβ), and fibroblast growth factor (FGF).

In some embodiments, the growth factor is vascular endothelial growth factor (VEGF), placenta derived growth factor (PDGF), transforming growth factor beta (TGFβ), fibroblast growth factor (FGF), or a combination thereof.

In some embodiments, the growth factor is vascular growth factor (VEGF).

In some embodiments, the exterior wall portion is coated with a hydrophobic polymer.

In some embodiments, the hydrophobic polymer is a siloxane polymer or a polycaprolactone (PCL).

In some embodiments, the implantable device further includes the multiple cells contained within the interior region of the container.

In some embodiments, the multiple cells are human beta cells.

In some embodiments, the therapeutic agent is an antibody or antibody fragment, an enzyme, an engineered protein, or a protein fragment.

In some embodiments, the engineered protein is coagulation factor VIIa, coagulation factor VIII, coagulation factor IX, follicle stimulating hormone (FSH), anti-thrombin III, erythropoietin, granulocyte colony stimulating factor (GCSF), granulocyte macrophage colony stimulating factor (GMCSF), insulin, glucagon-like peptide-1 (GLP-1), agalsidase beta, alpha-glucosidase, laronidase, sphingomyelinase, phenylalanine hydroxylase, interleukin 2 (IL-2), interleukin 11 (IL-11), interleukin 12 (IL-12), or vascular endothelial growth factor (VEGF).

In some embodiments, the engineered protein is a hormone, an enzyme, or a cytokine.

In some embodiments, the hormone is insulin or an insulin analog.

In some embodiments, the multiple cells are human beta cells, and the therapeutic agent is insulin.

In some embodiments, the substance is hydrogen peroxide, carbamide peroxide, magnesium peroxide, or calcium peroxide.

In some embodiments, the substance is magnesium peroxide.

In some embodiments, a generation rate of oxygen is between about 130 nL per minute per gram of magnesium peroxide.

In some embodiments, a volume of oxygen released is between about 30 µL per day.

In some embodiments, the implantable device further includes a carrier including the substance and multiple particles including a catalyst, wherein the multiple particles are part of a coating applied to an inner surface of the interior wall portion.

In some embodiments, the substance is hydrogen peroxide and the catalyst is catalase. In some embodiments, the implantable device further includes one or more additional containers associated with the container, wherein each of the one or more additional containers contains additional, multiple cells.

In some embodiments, the container and the one or more additional containers are spaced apart from one another.

In some embodiments, the implantable device further includes a flexible structure that maintains the container and the one or more additional containers in a spaced configuration.

In some embodiments, the container and the one or more additional containers are arranged in a matrix configuration.

In a second aspect, according to the present invention, a therapeutic system includes an accessory device and an implantable device according the present invention, the implantable device configured to be implanted subcutaneously along a skin region and to provide a therapeutic agent. The implantable device includes a container configured to contain multiple cells capable of producing the therapeutic agent within an interior region of the container. The container defines first pores defined by an interior wall portion of the container, the first pores having a first average size that (i) allows passage of the therapeutic agent through the first pores and (ii) prevents passage of immune cells through the first pores. The container further defines second pores defined by an exterior wall portion of the container, the second pores having a second average size that is larger than the first average size, and the second pores being sized to promote vascularization along the exterior wall portion. The implantable device further includes a substance capable of reacting to generate oxygen for the multiple cells within the interior region of the container. The accessory device is configured to cooperate with the implantable device for promoting delivery of oxygen to the multiple cells.

Embodiments, may include one or more of the following features.

In some embodiments, the substance is a first substance, wherein the accessory device includes a transdermal patch that is configured to be secured to an exterior surface of the skin region, and wherein the transdermal patch includes multiple microneedles that carry a second substance capable of reacting to generate oxygen for the multiple cells within the interior region of the container.

In some embodiments, the substance is hydrogen peroxide, carbamide peroxide, magnesium peroxide, or calcium peroxide.

In some embodiments, the first substance is magnesium peroxide.

In some embodiments, a generation rate of oxygen is between about 130 nL per minute per gram of magnesium peroxide.

In some embodiments, a volume of oxygen released is between about 30 µL per day.

In some embodiments, the accessory device includes a cuff that is configured to be secured to and apply pressure to an exterior surface of the skin region to direct a flow of blood towards the implantable device.

In some embodiments, the accessory device includes a sintered mesh that surrounds the implantable device and that carries a substance capable of reacting to generate oxygen.

In some embodiments, the multiple cells are human cells.

In some embodiments, the multiple cells are human beta cells.

In some embodiments, the therapeutic agent is an antibody or antibody fragment, an enzyme, an engineered protein, a protein fragment, or a peptide.

In some embodiments, the engineered protein is coagulation factor VIIa, coagulation factor VIII, coagulation factor IX, follicle stimulating hormone (FSH), anti-thrombin III, erythropoietin, granulocyte colony stimulating factor (GCSF), granulocyte macrophage colony stimulating factor (GMCSF), insulin, glucagon-like peptide-1 (GLP-1), agalsidase beta, alpha-glucosidase, laronidase, sphingomyelinase, phenylalanine hydroxylase, interleukin 2 (IL-2), interleukin 11 (IL-11), interleukin 12 (IL-12), or vascular endothelial growth factor (VEGF).

In some embodiments, the engineered protein is a hormone, an enzyme, or a cytokine.

In some embodiments, the hormone is insulin or an insulin analog.

In some embodiments, the multiple cells are human beta cells, and the therapeutic agent is insulin.

In a third aspect, according to the present invention, an implantable device for providing a therapeutic agent includes a container configured to contain multiple cells capable of producing the therapeutic agent within an interior region of the container. The container defines first pores defined by an interior wall portion of the container, the first pores having a first average size that (i) allows passage of the therapeutic agent through the first pores and (ii) prevents passage of immune cells through the first pores. The container further defines second pores defined by an exterior wall portion of the container, the second pores having a second average size that is larger than the first average size, and the second pores being sized to promote vascularization along the exterior wall portion. The implantable device further includes a substance capable of reacting to generate oxygen for the multiple cells within the interior region of the container.

Embodiments may include one or more of the following features.

In some embodiments, the second average size of the second pores locates vasculature along the second pores, and the second average size of the second pores allows passage of the therapeutic agent through the exterior wall portion to the vasculature.

In some embodiments, the first pores have first widths in a range of about 10 nm to about 400 nm, and wherein the second pores have second widths in a range of about 2 µm to about 60 µm.

In some embodiments, the interior region has a width that is in a range of about 100 µm to about 2 mm. In a not claimed comparative example, the interior region can be wide enough to accommodate multiple cells of the multiple cells across a plane that is perpendicular to a central axis of the container.

In some embodiments, the container has a wall thickness in a range of about 1 µm to about 100 µm.

In a not claimed comparative example, the container includes a tube, wherein the tube has a linear configuration. According to the present invention, the tube has a spiral configuration or a helical configuration.

In some embodiments, the implantable device further includes a second tube disposed inside of the first tube, wherein the first and second tubes together define an annular lumen for containing the multiple cells, wherein the implantable device includes multiple particles including the substance, and wherein the multiple particles are disposed within a core region of the second tube.

In some embodiments, the implantable device further includes multiple particles including the substance, wherein the multiple particles are part of a coating applied to an inner surface of the interior wall portion.

In some embodiments, the exterior wall portion is coated with a growth factor that promotes vascularization, and wherein the growth factor is covalently linked to the exterior wall portion, and/or wherein the growth factor is electrostatically bound to the exterior wall portion, and/or wherein the growth factor is site-specifically bound to the exterior wall portion, and/or wherein the growth factor is selected from the group consisting of: vascular endothelial growth factor (VEGF), placenta derived growth factor (PDGF), transforming growth factor beta (TGFβ), and fibroblast growth factor (FGF), and/or wherein the growth factor is vascular endothelial growth factor (VEGF), placenta derived growth factor (PDGF), transforming growth factor beta (TGFβ), fibroblast growth factor (FGF), or a combination thereof, and/or wherein the growth factor is vascular growth factor (VEGF).

In some embodiments, the implantable device further includes the multiple cells contained within the interior region of the container.

In some embodiments, the multiple cells are beta cells, and the therapeutic agent is insulin.

In some embodiments, the implantable device further includes one or more additional containers associated with the container, wherein each of the one or more additional containers contains additional, multiple cells.

In some embodiments, the container includes a tubular wall that includes the interior and exterior wall portions, wherein the tubular wall further defines third pores arranged radially between the first and second pores, wherein the third pores have a third average size that is greater than the first average size and less than the second average size, wherein pores defined by the tubular wall gradually increase in size from an interior surface of the tubular wall to an exterior surface of the tubular wall, and wherein the container includes one or more of polycaprolactone, PTFE, ePTFE, nylon, polyether-ketone, polyether sulfone, polyester, polyvinylidene difluoride, and polysiloxane.

In some embodiments, the interior wall portion includes a tubular member and the exterior wall portion includes a coating that surrounds the tubular member, wherein the tubular member has a first material formulation and the coating has a second material formulation that is different from the first material formulation, wherein the first material formulation includes one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups, and wherein the second material formulation includes one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In a fourth aspect, according to the present invention, a therapeutic system includes the implantable device of the third aspect, and the implantable device configured to be implanted subcutaneously along a skin region. The therapeutic system further includes an accessory device configured to cooperate with the implantable device for promoting delivery of oxygen to the multiple cells, wherein the accessory device includes (i) a transdermal patch that is configured to be secured to an exterior skin surface of the skin region, the transdermal patch including multiple needles that carry a substance capable of reacting to generate oxygen for the multiple cells within the interior volume of the container, or (ii) a cuff that is configured to be secured to and apply pressure to the exterior skin surface of the skin region to direct a flow of blood towards the implantable device, or (iii) a sintered mesh that surrounds the implantable device and that carries a substance capable of reacting to generate oxygen.

In a fifth aspect, an implantable device for providing a therapeutic agent includes a container configured to contain multiple cells capable of producing the therapeutic agent within an interior region of the container. The container defines first pores defined by an interior wall portion of the container, the first pores having a first average size that (i) allows passage of the therapeutic agent and oxygen through the first pores and (ii) prevents passage of immune cells through the first pores. The container further defines second pores defined by an exterior wall portion of the container, the second pores having a second average size that is larger than the first average size, and the second pores being sized to promote vascularization and oxygen transfer along the exterior wall portion.

Embodiments may include one or more of the following features.

In some embodiments, the implantable device has an external surface area to volume ratio of about 200 to about 5,000.

In some embodiments, the implantable device has an aspect ratio of about 100 to about 2,000.

In some embodiments, the container includes a tubular wall that includes the interior and exterior wall portions.

In some embodiments, the tubular wall further defines third pores arranged radially between the first and second pores, the third pores having a third average pore size that is greater than the first average pore size and less than the second average pore size.

In some embodiments, the pores defined by the tubular wall gradually increase in size from an interior surface of the tubular wall to an exterior surface of the tubular wall.

In some embodiments, the container includes one or more of polycaprolactone, PTFE, ePTFE, nylon, polyether-ketone, polyether sulfone, polyester, polyvinylidene difluoride, and polysiloxane.

In some embodiments, the interior wall portion includes a tubular member, and the exterior wall portion includes a coating that surrounds the tubular member.

In some embodiments, the tubular member has a first material formulation, and the coating has a second material formulation that is different from the first material formulation.

In some embodiments, the first material formulation includes one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups.

In some embodiments, the second material formulation includes one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In some embodiments, the second average size of the second pores locates vasculature along the second pores.

In some embodiments, the second average size of the second pores allows passage of the therapeutic agent through the exterior wall portion to vasculature adjacent the implantable device.

In some embodiments, the first pores have widths in a range of about 10 nm to about 400 nm.

In some embodiments, the second pores have widths in a range of about 2 µm to about 60 µm.

According to the present invention, the interior region has a width that is limited to accommodating a single cell of the multiple cells.

In some embodiments, the width is in a range of about 100 µm to about 2 mm.

In a not claimed comparative example, the interior region can be wide enough to accommodate several of the multiple cells across a plane that is perpendicular to a central axis of the container.

In some embodiments, the container has a wall thickness in a range of about 1 µm to about 100 µm.

In some embodiments, the implantable device has a generally tubular shape.

In a not claimed comparative example, the implantable device includes a tube that has a linear configuration. According to the present invention, the tube that has a spiral configuration or a helical configuration.

In some embodiments, ends of the tube are sealed.

In some embodiments, the tube is a first tube, and the implantable device further includes a second tube disposed inside of the first tube, wherein the first and second tubes together define an annular lumen for containing the multiple cells.

In some embodiments, the exterior wall portion is coated with a growth factor that promotes vascularization.

In some embodiments, the growth factor is covalently linked to the exterior wall portion.

In some embodiments, the growth factor is electrostatically bound to the exterior wall portion.

In some embodiments, the growth factor is site-specifically bound to the exterior wall portion.

In some embodiments, the growth factor is selected from the group consisting of: vascular endothelial growth factor (VEGF), placenta derived growth factor (PDGF), transforming growth factor beta (TGFβ), and fibroblast growth factor (FGF).

In some embodiments, the growth factor is vascular endothelial growth factor (VEGF), placenta derived growth factor (PDGF), transforming growth factor beta (TGFβ), fibroblast growth factor (FGF), or a combination thereof.

In some embodiments, the growth factor is vascular endothelial growth factor (VEGF).

In some embodiments, the implantable device further includes the multiple cells contained within the interior region of the container.

In some embodiments, the multiple cells are human beta cells.

In some embodiments, the therapeutic agent is an antibody or an antibody fragment, an enzyme, an engineered protein, a protein fragment, or a peptide.

In some embodiments, the engineered protein is coagulation factor VIIa, coagulation factor VIII, coagulation factor IX, follicle stimulating hormone (FSH), anti-thrombin III, erythropoietin, granulocyte colony stimulating factor (GCSF), granulocyte macrophage colony stimulating factor (GMCSF), insulin, glucagon-like peptide-1 (GLP-1), agalsidase beta, alpha-glucosidase, laronidase, sphingomyelinase, phenylalanine hydroxylase, interleukin 2 (IL-2), interleukin 11 (IL-11), interleukin 12 (IL-12), or vascular endothelial growth factor (VEGF).

In some embodiments, the engineered protein is a hormone, an enzyme, or a cytokine.

In some embodiments, the hormone is insulin or an insulin analog.

In some embodiments, the multiple cells are beta cells, and the therapeutic agent is insulin.

In some embodiments, the container is a first container that contains the multiple cells, wherein the implantable device further includes one or more additional containers associated with the first container, and wherein each of the one or more additional containers contains additional multiple cells.

In some embodiments, the first container and the one or more additional containers are spaced apart from one another.

In some embodiments, the implantable device further includes a flexible structure that maintains the first container and the one or more additional containers in a spaced configuration.

In some embodiments, the first container and the one or more additional containers are arranged in a matrix configuration.

In a sixth aspect, according to the present invention, a therapeutic system includes an accessory device and an implantable device according to the present invention, the implantable device configured to be implanted subcutaneously along a skin region and to provide a therapeutic agent. The implantable device includes a container configured to contain multiple cells capable of producing the therapeutic agent within an interior region of the container. The container defines first pores defined by an interior wall portion of the container, the first pores having a first average size that (i) allows passage of the therapeutic agent and oxygen through the first pores and (ii) prevents passage of immune cells through the first pores. The container further defines second pores defined by an exterior wall portion of the container, the second pores having a second average size that is larger than the first average size, and the second pores being sized to promote vascularization and oxygen transfer along the exterior wall portion.

Embodiments may include one or more of the following features.

In some embodiments, the accessory device includes a transdermal patch that is configured to be secured to an exterior surface of the skin region, the transdermal patch including multiple microneedles that carry a substance capable of reacting to generate oxygen for the multiple cells within the interior region of the container.

In some embodiments, the substance is hydrogen peroxide, carbamide peroxide, magnesium peroxide, or calcium peroxide.

In some embodiments, the substance is magnesium peroxide.

In some embodiments, a generation rate of oxygen is between about 130 nL per minute per gram of magnesium peroxide.

In some embodiments, a volume of oxygen released is between about 30 µL per day.

In some embodiments, the accessory device includes a cuff that is configured to be secured to and to apply pressure to the skin region to direct a flow of blood towards the implantable device.

In some embodiments, the accessory device includes a sintered mesh that surrounds the implantable device and that carries a substance capable of reacting to generate oxygen.

In some embodiments, the multiple cells are human cells.

In some embodiments, the multiple cells are human beta cells.

In some embodiments, the therapeutic agent is an antibody or an antibody fragment, an enzyme, an engineered protein, a protein fragment, or a peptide.

In some embodiments, the engineered protein is coagulation factor VIIa, coagulation factor VIII, coagulation factor IX, follicle stimulating hormone (FSH), anti-thrombin III, erythropoietin, granulocyte colony stimulating factor (GCSF), granulocyte macrophage colony stimulating factor (GMCSF), insulin, glucagon-like peptide-1 (GLP-1), agalsidase beta, alpha-glucosidase, laronidase, sphingomyelinase, phenylalanine hydroxylase, interleukin 2 (IL-2), interleukin 11 (IL-11), interleukin 12 (IL-12), or vascular endothelial growth factor (VEGF).

In some embodiments, the engineered protein is a hormone, an enzyme, or a cytokine.

In some embodiments, the hormone is insulin or an insulin analog.

In some embodiments, the multiple cells are human beta cells, and the therapeutic agent is insulin.

In a seventh aspect, according to the present invention, an implantable device for providing a therapeutic agent includes a container configured to contain multiple cells capable of producing the therapeutic agent within an interior region of the container. The container defines first pores defined by an interior wall portion of the container, the first pores having a first average size that (i) allows passage of the therapeutic agent and oxygen through the first pores and (ii) prevents passage of immune cells through the first pores. The container further defines second pores defined by an exterior wall portion of the container, the second pores having a second average size that is larger than the first average size, and the second pores being sized to promote vascularization and oxygen transfer along the exterior wall portion.

Embodiments may include one or more of the following features.

In some embodiments, the implantable device has an external surface area to volume ratio of about 200 to about 5,000.

In some embodiments, the second average size of the second pores locates vasculature along the second pores, and the second average size of the second pores allows passage of the therapeutic agent through the exterior wall portion to vasculature adjacent the implantable device.

In some embodiments, the first pores have first widths in a range of about 10 nm to about 400 nm, and wherein the second pores have second widths in a range of about 2 µm to about 60 µm.

In some embodiments, the container has a wall thickness in a range of 1 µm to 100 µm. According to the present invention, the interior region has a width that is limited to accommodating a single cell of the multiple cells. The width may be in a range of about 100 µm to about 2 mm. In a not claimed comparative example, the interior region can be wide enough to accommodate multiple of the cells across a plane that is perpendicular to a central axis of the container.

In a not claimed comparative example, the container includes a tube, wherein the tube has a linear configuration. According to the present invention, the tube has a spiral configuration or a helical configuration.

In some embodiments, the tube is a first tube, wherein the implantable device includes a second tube disposed inside of the first tube, and wherein the first and second tubes together define an annular lumen for containing the multiple cells.

In some embodiments, the exterior wall portion is coated with a growth factor that promotes vascularization, and wherein the growth factor is covalently linked to the exterior wall portion, and/or wherein the growth factor is electrostatically bound to the exterior wall portion, and/or wherein the growth factor is site-specifically bound to the exterior wall portion, and/or wherein the growth factor is selected from the group consisting of: vascular endothelial growth factor (VEGF), placenta derived growth factor (PDGF), transforming growth factor beta (TGFβ), and fibroblast growth factor (FGF), and/or wherein the growth factor is vascular endothelial growth factor (VEGF), placenta derived growth factor (PDGF), transforming growth factor beta (TGFβ), fibroblast growth factor (FGF), or a combination thereof, and/or wherein the growth factor is vascular growth factor (VEGF).

In some embodiments, the implantable device further includes the muliple cells contained within the interior region of the container.

In some embodiments, the multiple cells are beta cells, and the therapeutic agent is insulin.

In some embodiments, the implantable device further includes one or more additional containers associated with the container, wherein each of the one or more additional containers contains additional, multiple cells.

In some embodiments, the container includes a tubular wall that includes the interior and exterior wall portions, wherein the tubular wall further defines third pores arranged radially between the first and second pores, wherein the third pores have a third average size that is greater than the first average size and less than the second average size, wherein pores defined by the tubular wall gradually increase in size from an interior surface of the tubular wall to an exterior surface of the tubular wall, and wherein the container includes one or more of polycaprolactone, PTFE, ePTFE, nylon, polyether-ketone, polyether sulfone, polyester, polyvinylidene difluoride, and polysiloxane.

In some embodiments, the interior wall portion includes a tubular member and the exterior wall portion includes a coating that surrounds the tubular member, wherein the tubular member has a first material formulation and the coating has a second material formulation that is different from the first material formulation, wherein the first material formulation includes one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups, and wherein the second material formulation includes one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In an eighth aspect, according to the present invention, a therapeutic system includes the implantable device of the seventh aspect, the implantable device configured to be implanted subcutaneously along a skin region. The therapeutic system further includes an accessory device configured to cooperate with the implantable device for promoting delivery of oxygen to the multiple cells.

Embodiments may include one or more of the following features.

In some embodiments, the accessory device includes (i) a transdermal patch that is configured to be secured to an exterior skin surface of the skin region, the transdermal patch including multiple of needles that carry a substance capable of reacting to generate oxygen for the multiple cells within the interior volume of the container, or (ii) a cuff that is configured to be secured to and apply pressure to the exterior skin surface of the skin region to direct a flow of blood towards the implantable device, or (iii) a sintered mesh that surrounds the implantable device and that carries a substance capable of reacting to generate oxygen.

In a ninth aspect, a not claimed method of manufacturing a therapeutic device includes providing a member that defines first pores having a first average size that (i) allows passage of a therapeutic agent through the first pores and (ii) prevents passage of immune cells through the first pores. The method further includes spinning the member about an axis of the member. The method further includes applying a fibrous material to the member while the member spins to form a coating that surrounds the member, the coating defining second pores having a second average size that is larger than the first average size, and the second pores being sized to promote vascularization along the coating. The member and the coating together form a container of the therapeutic device, the container defining an interior region for accommodating multiple cells capable of producing the therapeutic agent.

Examples may include one or more of the following features.

In some examples, the method further includes adhering the coating to the member.

In some examples, the method further includes forming the member as a tubular wall using an extrusion apparatus.

In some examples, the method further includes performing an electrospinning process to apply the fibrous material to the member.

In some examples, the member includes one or more materials including expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and and polymers containing carboxybetaine, sulfobetaine, and phosphoryl choline groups.

In some examples, the therapeutic device has an external surface area to volume ratio of about 200 to about 5,000.

In some examples, the fibrous material includes one or more materials including polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In some examples, the first pores have widths in a range of about 10 nm to about 400 nm.

In some examples, the second pores have widths in a range of about 2 µm about to 60 µm.

In some examples, the container has a wall thickness in a range of about 1 µm to about 100 µm.

In some examples, the method further includes loading the member onto a support member prior to spinning the member about the axis.

In some examples, the method further includes terminating spinning of the member, and removing the container from the support member.

In some examples, the method further includes sealing a first end of the container, inserting the multiple cells into the interior region of the container, and sealing a second end of the container to form the therapeutic device, the second end being opposite from the first end.

According to the present invention, the interior region has a width that is limited to accommodating a single cell of the multiple cells.

In some examples, the width is in a range of about 100 µm to about 2 mm.

In some examples, the multiple cells are beta cells, and the therapeutic agent is insulin.

In some examples, the support member includes a tubular mandrel such that the container forms a tube.

In some examples, the method further includes maintaining the tube in a linear configuration according to a not claimed comparative example. In some examples, the method further includes deforming the tube into a spiral configuration or deforming the tube into a helical configuration, according to the present invention.

In some examples, the method further includes applying a growth factor to the coating that promotes vascularization.

In some examples, the method further includes forming one or more additional containers.

In some examples, the method further includes associating the container with the one or more additional containers in a spaced configuration with a flexible structure that maintains the spaced configuration.

In some examples, the method further includes coupling the container to the one or more additional containers in a matrix configuration.

In some examples, the method further includes loading the container with a substance capable of reacting to generate oxygen within the interior region for the multiple cells.

In some examples, the method further includes applying multiple particles including the substance to an inner surface of the member.

In some examples, the member is a first tubular wall, wherein the method further includes positioning a second tubular wall interiorly to the first tubular wall, and wherein the first and second tubular walls together define an annular lumen for accommodating the multiple cells.

In some examples, the method further includes applying the substance to a core region of the second tubular wall.

In a tenth aspect, a not claimed method of manufacturing a therapeutic device includes providing a member that defines first pores having a first average size that (i) allows passage of a therapeutic agent through the first pores and (ii) prevents passage of immune cells through the first pores. The method further includes spinning the member about an axis of the member. The method further includes applying a fibrous material to the member while the member spins to form a coating that surrounds the member, the coating defining second pores having a second average size that is larger than the first average size, and the second pores being sized to promote vascularization along the coating. The member and the coating together form a container of the therapeutic device, the container defining an interior region for accommodating multiple cells capable of producing the therapeutic agent.

Examples may include one or more of the following features.

In some examples, the method further includes adhering the coating to the member.

In some examples, the method further includes forming the member as a tubular wall using an extrusion apparatus.

In some examples, the method further includes performing an electrospinning process to apply the fibrous material to the member.

In some examples, the member includes one or more first materials including expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups.

In some examples, the fibrous material includes one or more second materials including polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In some examples, the first pores have first widths in a range of about 10 nm to about 400 nm, and wherein the second pores have second widths in a range of about 2 µm to about 60 µm, and wherein the container has a wall thickness in a range of 1 µm to 100 µm.

In some examples, the therapeutic device has an external surface area to volume ratio of about 200 to about 5,000.

In some examples, the method further includes loading the member onto a support member prior to spinning the member about the axis, terminating spinning of the member, removing the container from the support member, sealing a first end of the container, inserting the multiple cells into the interior region of the container, and sealing a second end of the container to form the therapeutic device, the second end being opposite from the first end.

According to the present invention, the interior region has a width that is limited to accommodating a single cell of the multiple cells. The width of the interior region may be in a range of about 100 µm to about 2 mm.

In some examples, the multiple cells are beta cells, and the therapeutic agent is insulin.

In some examples, the support member includes a tubular mandrel such that the container forms a tube, and the method further includes maintaining the tube in a linear configuration according to a not claimed comparative example, or the method further includes deforming the tube into a spiral configuration or deforming the tube into a helical configuration according to the present invention.

In some examples, the method further includes applying a growth factor to the coating, the growth factor promoting vascularization.

In some examples, the method further includes forming one or more additional containers, and associating the container with the one or more additional containers in a spaced configuration with a flexible structure that maintains the spaced configuration or coupling the container to the one or more additional containers in a matrix configuration.

In some examples, the method further includes loading the container with a substance capable of reacting to generate oxygen within the interior region for the multiple cells; and (i) applying first multiple particles including the substance to an inner surface of the member, or (ii) positioning a tubular wall interiorly to the member, wherein the tubular wall and the member together define an annular lumen for accommodating the multiple cells, and applying the substance to a core region of the tubular wall.

In an eleventh aspect, a not claimed method of manufacturing a therapeutic device includes forming a container wall that is sized to contain multiple cells capable of producing a therapeutic agent within an interior region of the container wall and exposing the container wall to a solvent to remove material from the container wall to define first pores along an interior portion of the container wall and second pores along an exterior portion of the container wall. The first pores have a first average size that (i) allows passage of the therapeutic agent through the first pores and (ii) prevents passage of immune cells through the first pores. The second pores have a second average size that is larger than the first average size, and the second pores are sized to promote vascularization along the exterior portion.

Examples may include one or more of the following features.

In some examples, exposing the container wall includes carrying out a non-solvent induced phase separation (NIPS) technique.

In some examples, the solvent includes one or more of noprotic solvents of different polarities, such as N-methyl pyrrolidone, N,N-dimethyl acetamide, tetrahydrofuran, toluene, DMF, tetramethyl urea, methyl ethyl ketone, and supercritical CO2.

In some examples, the method further includes increasing an exposure time to increase sizes of the first pores and the second pores.

In some examples, forming the container wall includes rolling a sheet of material into a tube.

In some examples, removing the material from the container wall further defines third pores with an average third size that gradually increases from the first average pore size along the interior portion to the second average pore size along the exterior portion.

In some examples, the container wall includes one or more of polycaprolactone, PTFE, ePTFE, nylon, polyether-ketone, polyether sulfone, polyester, polyvinylidene difluoride, and polysiloxane.

In some examples, the first pores have widths in a range of about 10 nm to about 400 nm.

In some examples, the second pores have widths in a range of about 2 µm to about 60 µm.

In some examples, the container wall has a wall thickness in a range of about 1 µm to about 100 µm.

In some examples, the method further includes sealing a first end of the container wall, inserting the multiple cells into the interior region of the container wall, and sealing a second end of the container wall to form the therapeutic device, the second end being opposite from the first end.

According to the present invention, the interior region has a width that is limited to accommodating a single cell of the multiple cells.

In some examples, the width is in a range of about 100 µm to 2 mm.

In some examples, the multiple cells are beta cells, and the therapeutic agent is insulin.

In some examples, the container wall includes a tube.

In some examples, the method further includes maintaining the tube in a linear configuration according to not claimed comparative example. In some examples, the method further includes deforming the tube into a spiral configuration or deforming the tube into a helical configuration according to the present invention.

In some examples, the method further includes coating the exterior portion of the container wall with a growth factor that promotes vascularization.

In some examples, the method further includes forming one or more additional container walls.

In some examples, the method further includes associating the container wall with the one or more additional container walls in a spaced configuration with a flexible structure that maintains the spaced configuration.

In some examples, the method further includes coupling the container wall to the one or more additional container walls in a matrix configuration.

In some examples, the method further includes loading the container wall with a substance capable of reacting to generate oxygen within the interior region for the multiple cells.

In some examples, the method further includes applying multiple particles including the substance to an inner surface of the container wall.

In some examples, the container wall is a first tube, wherein the method further includes positioning a second tube interiorly to the first tube, and wherein the first and second tubes together define an annular lumen for accommodating the multiple cells.

In some examples, the method further includes applying the substance to a core region of the second tube.

In some examples, the implantable device has an external surface area to volume ratio of about 200 to about 5,000.

In a twelfth aspect, a not claimed method of manufacturing a therapeutic device includes forming a container wall that is sized to contain multiple cells capable of producing a therapeutic agent within an interior region of the container wall and exposing the container wall to a solvent to remove material from the container wall to define first pores along an interior portion of the container wall and second pores along an exterior portion of the container wall. The first pores have a first average size that (i) allows passage of the therapeutic agent through the first pores and (ii) prevents passage of immune cells through the first pores. The second pores have a second average size that is larger than the first average size, and the second pores are sized to promote vascularization along the exterior portion.

Examples may include one or more of the following features.

In some examples, exposing the container wall includes carrying out a non-solvent induced phase separation (NIPS) technique, and wherein the solvent includes one or more of noprotic solvents of different polarities, such as N-methyl pyrrolidone, N,N-dimethyl acetamide, tetrahydrofuran, toluene, DMF, tetramethyl urea, methyl ethyl ketone, and supercritical CO2, and wherein the method further includes increasing an exposure time to increase sizes of the first pores and second pores.

In some examples, removing the material from the container wall further defines third pores with an average third size that gradually increases from the first average pore size along the interior portion to the second average pore size along the exterior portion.

In some examples, forming the container wall includes rolling a sheet of material into a tube, and the container wall includes one or more of polycaprolactone, PTFE, ePTFE, nylon, polyether-ketone, polyether sulfone, polyester, polyvinylidene difluoride, and polysiloxane.

In some examples, the first pores have first widths in a range of about 10 nm to about 400 nm, and the second pores have second widths in a range of about 2 µm to about 60 µm.

In some examples, the container wall has a wall thickness in a range of about 1 µm to about 100 µm.

In some examples, the therapeutic device has an external surface area to volume ratio of about 200 to about 5,000.

In some examples, the method further includes sealing a first end of the container wall, inserting the multiple cells into the interior region of the container wall, and sealing a second end of the container wall to form the therapeutic device, the second end being opposite from the first end.

According to the present invention, the interior region has a width that is limited to accommodating a single cell of the multiple cells. The width may be in a range of about 100 µm to about 2 mm.

In some examples, the multiple cells are beta cells, and the therapeutic agent is insulin.

In some examples, the container wall includes a tube, and the method further includes maintaining the tube in a linear configuration according to a not claimed comparative example. In some examples, the method further includes deforming the tube into a spiral configuration or deforming the tube into a helical configuration according to the present invention.

In some examples, the method further includes coating the exterior portion of the container wall with a growth factor that promotes vascularization.

In some examples, the method further includes forming one or more additional container walls and associating the container wall with the one or more additional container walls in a spaced configuration or coupling the container wall to the one or more additional container walls in a matrix configuration.

In some examples, the method further includes loading the container wall with a substance capable of reacting to generate oxygen within the interior region for the multiple cells, and/or applying multiple particles including the substance to an inner surface of the container wall.

In some examples, the container wall is a first tube, wherein the method further includes positioning a second tube interiorly to the first tube, wherein the first and second tubes together define an annular lumen for accommodating the multiple cells, and wherein the method further includes applying the substance to a core region of the second tube.

In a thirteenth aspect, a not claimed method of treating a disease in a subject, the method including implanting an implantable device subcutaneously into the subject, wherein a substance of the implantable device generates oxygen for use by multiple cells contained in an interior region of a container of the implantable device, wherein a therapeutic agent generated by the multiple cells contained in the interior region of the container passes through the container, and wherein the container of the implantable device prevents immune cells from passing from surrounding vasculature and/or tissue into the interior region of the container containing the multiple cells.

Examples may include one or more of the following features.

In some examples, the container defines first pores defined by an interior wall portion of the container, the first pores having a first average size that (i) allows passage of the therapeutic agent through the first pores and (ii) prevents passage of the immune cells through the first pores. The container further definessecond pores defined by an exterior wall portion of the container, the second pores having a second average size that is larger than the first average size, the second pores being sized to promote vascularization along the exterior wall portion, and the second pores being sized to locate vasculature along the second pores.

In some examples, the container includes a tubular wall that includes the interior and exterior wall portions.

In some examples, the tubular wall further defines third pores arranged radially between the first and second pores, the third pores having a third average size that is greater than the first average size and less than the second average size.

In some examples, the pores defined by the tubular wall gradually increase in size from an interior surface of the tubular wall to an exterior surface of the tubular wall.

In some examples, the container includes one or more of polycaprolactone, PTFE, ePTFE, nylon, polyether-ketone, polyether sulfone, polyester, polyvinylidene difluoride, and polysiloxane.

In some examples, the interior wall portion includes a tubular member and the exterior wall portion includes a coating that surrounds the tubular member.

In some examples, the tubular member has a first material formulation and the coating has a second material formulation that is different from the first material formulation.

In some examples, the first material formulation includes one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups.

In some examples, the second material formulation includes one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In some examples, the second average size of the second pores allows passage of the therapeutic agent through the exterior wall portion to vasculature adjacent the implantable device.

In some examples, the first pores have widths in a range of about 10 nm to about 400 nm.

In some examples, the second pores have widths in a range of about 2 µm to about 60 µm.

In some examples, the interior region has a width in a range of about 100 µm to about 2 mm.

In a not claimed comparative example, the interior region can be wide enough to accommodate multiple cells of the multiple cells across a plane that is perpendicular to a central axis of the container.

In some examples, the container has a wall thickness in a range of 1 µm to 100 µm.

In some examples, the implantable device has a generally tubular shape.

In some examples, the implantable device includes a tube, wherein, in a not claimed comparative example, the tube has a linear configuration, and wherein, according to the present invention, the tube has a spiral configuration or a helical configuration.

In some examples, the implantable device has an external surface area to volume ratio of about 200 to about 5,000.

In some examples, ends of the tube are sealed.

In some examples, the tube is a first tube, wherein the implantable device further includes a second tube disposed inside of the first tube, and wherein the first and second tubes together define an annular lumen for containing the multiple cells.

In some examples, the substance is disposed within a core region of the second tube.

In some examples, the implantable device further includes multiple particles including the substance, and wherein the multiple particles are part of a coating applied to an inner surface of the interior wall portion.

In some examples, the exterior wall portion is coated with a growth factor that promotes vascularization.

In some examples, the growth factor is covalently linked to the exterior wall portion.

In some examples, the growth factor is electrostatically bound to the exterior wall portion.

In some examples, the growth factor is site-specifically bound to the exterior wall portion.

In some examples, the growth factor is selected from the group consisting of: vascular endothelial growth factor (VEGF), placenta derived growth factor (PDGF), transforming growth factor beta (TGFβ), and fibroblast growth factor (FGF).

In some examples, the growth factor is vascular endothelial growth factor (VEGF), placenta derived growth factor (PDGF), transforming growth factor beta (TGFβ), fibroblast growth factor (FGF), or a combination thereof.

In some examples, the growth factor is vascular endothelial growth factor (VEGF).

In some examples, the exterior wall portion is coated with a hydrophobic polymer.

In some examples, the hydrophobic polymer is a siloxane polymer or a polycaprolactone (PCL).

In some examples, the implantable device further includes the multiple cells contained within the interior region of the container.

In some examples, the multiple cells are human beta cells.

In some examples, the therapeutic agent is an antibody or an antibody fragment, an enzyme, an engineered protein, or a protein fragment.

In some examples, the engineered protein is coagulation factor VIIa, coagulation factor VIII, coagulation factor IX, follicle stimulating hormone (FSH), anti-thrombin III, erythropoietin, granulocyte colony stimulating factor (GCSF), granulocyte macrophage colony stimulating factor (GMCSF), insulin, glucagon-like peptide-1 (GLP-1), agalsidase beta, alpha-glucosidase, laronidase, sphingomyelinase, phenylalanine hydroxylase, interleukin 2 (IL-2), interleukin 11 (IL-11), interleukin 12 (IL-12), or vascular endothelial growth factor (VEGF).

In some examples, the engineered protein is a hormone, and enzyme, or a cytokine.

In some examples, the hormone is insulin or an insulin analog.

In some examples, the multiple cells are beta cells, and the therapeutic agent is insulin.

In some examples, the substance is hydrogen peroxide, carbamide peroxide, magnesium peroxide, or calcium peroxide.

In some examples, the substance is magnesium peroxide.

In some examples, a generation rate of oxygen is about 130 nL per minute per gram of magnesium peroxide.

In some examples, a volume of oxygen released is about 30 µL per day.

In some examples, the generation of oxygen results in a flow rate of about 20 nL per minute.

In some examples, the implantable device further includes multiple particles including a catalyst, wherein the multiple particles are part of a coating applied to an inner surface of the interior wall portion.

In some examples, the catalyst is catalase.

In some examples, the container is a first container that contains the multiple cells, wherein the implantable device further includes one or more additional containers associated with the first container, and wherein each of the one or more additional containers contains additional, multiple cells.

In some examples, the first container and the one or more additional containers are spaced apart from one another.

In some examples, the implantable device further includes a flexible structure that maintains the first container and the one or more additional containers in a spaced configuration.

In some examples, the first container and the one or more additional containers are arranged in a matrix configuration.

In some examples, the implantable device is implanted into an arm or an abdomen of the subject.

In some examples, a body temperature of the subject at a time of implantation is between about 36 degrees Celsius and about 41 degrees Celsius.

In some examples, oxygen is generated and delivered at a sustained and controlled rate for between about 1 week to about 4 weeks.

In some examples, oxygen is generated and delivered at a sustained and controlled rate for between about 2 weeks to about 3 weeks.

In some examples, oxygen is generated and delivered at a sustained and controlled rate for between about 1 year to about 6 years.

In some examples, oxygen is generated and delivered at a sustained and controlled rate for about 2 to about 3 years.

In some examples, the subject has type 1 diabetes.

In some examples, the subject has hemophilia.

In a fourteenth aspect, according to the present invention, an implantable device for providing a therapeutic agent for use in the treatment of a disease includes a container configured to contain multiple cells capable of producing the therapeutic agent within an interior region of the container. The container defines first pores defined by an interior wall portion of the container, the first pores having a first average size that (i) allows passage of the therapeutic agent through the first pores and (ii) prevents passage of immune cells through the first pores. The container further defines second pores defined by an exterior wall portion of the container, the second pores having a second average size that is larger than the first average size, and the second pores being sized to promote vascularization along the exterior wall portion. The implantable device further includes a substance capable of reacting to generate oxygen for the multiple cells within the interior region of the container, wherein the implantable device is to be administered to a patient in need thereof.

Embodiments may include one or more of the following features.

In some embodiments, the second average size of the second pores locates vasculature along the second pores, and wherein the second average size of the second pores allows passage of the therapeutic agent through the exterior wall portion to the vasculature.

In some embodiments, the first pores have first widths in a range of about 10 nm to about 400 nm, wherein the second pores have second widths in a range of about 2 µm to about 60 µm, and wherein the container has a wall thickness in a range of about 1 µm to about 100 µm.

In some embodiments, the interior region has a width in a range of about 100 µm to about 2 mm. In a not claimed comparative example, the interior region can be wide enough to accommodate multiple cells of the multiple cells across a plane that is perpendicular to a central axis of the container.

In some embodiments, the container includes a tube, wherein, in a not claimed comparative example, the tube has a linear configuration, and wherein, according to the present invention, the tube has a spiral configuration or a helical configuration.

In some embodiments, the tube is a first tube, wherein the implantable device further includes a second tube disposed inside of the first tube, wherein the first and second tubes together define an annular lumen for containing the multiple cells, and wherein the substance is disposed within a core region of the second tube.

In some embodiments, the implantable device further includes multiple particles including the substance, wherein the particles are part of a coating applied to an inner surface of the interior wall portion.

In some embodiments, the exterior wall portion is coated with a growth factor that promotes vascularization, and wherein the growth factor is covalently linked to the exterior wall portion, and/or wherein the growth factor is electrostatically bound to the exterior wall portion, and/or wherein the growth factor is site-specifically bound to the exterior wall portion, and/or wherein the growth factor is selected from the group consisting of: vascular endothelial growth factor (VEGF), placenta derived growth factor (PDGF), transforming growth factor beta (TGFβ), and fibroblast growth factor (FGF), and/or wherein the growth factor is vascular endothelial growth factor (VEGF), placenta derived growth factor (PDGF), transforming growth factor beta (TGFβ), fibroblast growth factor (FGF), or a combination thereof, and/or wherein the growth factor is vascular growth factor (VEGF).

In some embodiments, the implantable device further includes the multiple cells contained within the interior region of the container.

In some embodiments, the multiple cells are beta cells, and the therapeutic agent is insulin.

In some embodiments, the implantable device further includes one or more additional containers associated with the container, wherein each of the one or more additional containers contains additional, multiple cells.

In some embodiments, the patient has type 1 diabetes or hemophilia.

In some embodiments, the container includes a tubular wall that includes the interior and exterior wall portions, wherein the tubular wall further defines third pores arranged radially between the first and second pores, wherein the third pores have a third average size that is greater than the first average size and less than the second average size, wherein pores defined by the tubular wall gradually increase in size from an interior surface of the tubular wall to an exterior surface of the tubular wall, and wherein the container includes one or more of polycaprolactone, PTFE, ePTFE, nylon, polyether-ketone, polyether sulfone, polyester, polyvinylidene difluoride, and polysiloxane.

In some embodiments, the interior wall portion includes a tubular member and the exterior wall portion includes a coating that surrounds the tubular member, wherein the tubular member has a first material formulation and the coating has a second material formulation that is different from the first material formulation, wherein the first material formulation includes one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups, and wherein the second material formulation includes one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In a fifteenth aspect, according to the present invention, a therapeutic system for use in the treatment of a disease includes the implantable device of the fourteenth aspect, the implantable device configured to be implanted subcutaneously along a skin region. The therapeutic system further includes an accessory device configured to cooperate with the implantable device for promoting delivery of oxygen to the multiple cells, wherein the therapeutic system is to be administered to a patient in need thereof.

In a sixteenth aspect, a not claimed method of treating a disease in a subject includes implanting an implantable device subcutaneously into the subject such that oxygen passes from surrounding vasculature into an interior region of a container of the implantable device, wherein a therapeutic agent generated by multiple cells contained in the interior region of the container passes through the container, and wherein the container of the implantable device prevents immune cells from passing from the surrounding vasculature and/or a surrounding tissue into the interior region of the container containing the multiple cells.

Examples may include one or more of the following features.

In some examples, the container defines first pores defined by an interior wall portion of the container, the first pores having a first average size that (i) allows passage of the therapeutic agent and oxygen through the first pores and (ii) prevents passage of the immune cells through the first pores. The container further defines second pores having a second average size that is larger than the first average size, the second pores being sized to promote vascularization and oxygen transfer along the exterior wall portion, and the second pores being sized to locate vasculature along the second pores.

In some examples, the container includes a tubular wall that includes the interior and exterior wall portions.

In some examples, the tubular wall further defines third pores arranged radially between the first and second pores, the third pores having a third average size that is greater than the first average size and less than the second average size.

In some examples, pores defined by the tubular wall gradually increase in size from an interior surface of the tubular wall to an exterior surface of the tubular wall.

In some examples, the container includes one or more of polycaprolactone, PTFE, ePTFE, nylon, polyether-ketone, polyether sulfone, polyester, polyvinylidene difluoride, and polysiloxane.

In some examples, the interior wall portion includes a tubular member and the exterior wall portion includes a coating that surrounds the tubular member.

In some examples, the tubular member has a first material formulation and the coating has a second material formulation that is different from the first material formulation.

In some examples, the first material formulation includes one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups.

In some examples, the second material formulation includes one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In some examples, the second average size of the second pores allows passage of the therapeutic agent through the exterior wall portion to vasculature adjacent the implantable device.

In some examples, the first pores have widths in a range of about 10 nm to 400 nm.

In some examples, the second pores have widths in a range of about 2 µm to about 60 µm.

According to the present invention, the interior region has a width that is limited to accommodating a single cell of the multiple cells.

In some examples, the width is in a range of about 100 µm to about 2 mm.

In some examples, the container has a wall thickness in a range of about 1 µm to about 100 µm.

In some examples, the implantable device has a generally tubular shape.

In some examples, the implantable device includes a tube, wherein, in a not claimed comparative example, the tube has a linear configuration, and wherein, according to the present invention, the tube has a spiral configuration or a helical configuration.

In some examples, the implantable device has an external surface area to volume ratio of about 200 to about 5,000.

In some examples, ends of the tube are sealed.

In some examples, the tube is a first tube, wherein the implantable device further includes a second tube disposed inside of the first tube, and wherein the first and second tubes together define an annular lumen for containing the multiple cells.

In some examples, the exterior wall portion is coated with a growth factor that promotes vascularization.

In some examples, the growth factor is covalently linked to the exterior wall portion.

In some examples, the growth factor is electrostatically bound to the exterior wall portion.

In some examples, the growth factor is site-specifically bound to the exterior wall portion.

In some examples, the growth factor is selected from the group consisting of: vascular endothelial growth factor (VEGF), placenta derived growth factor (PDGF), transforming growth factor beta (TGFβ), and fibroblast growth factor (FGF).

In some examples, the growth factor is vascular endothelial growth factor (VEGF), placenta derived growth factor (PDGF), transforming growth factor beta (TGFβ), fibroblast growth factor (FGF), or a combination thereof.

In some examples, the growth factor is vascular endothelial growth factor (VEGF).

In some examples, the implantable device further includes the multiple cells contained within the interior region of the container.

In some examples, the multiple cells are human beta cells.

In some examples, the therapeutic agent is an antibody or an antibody fragment, an enzyme, an engineered protein, or a protein fragment.

In some examples, the engineered protein is coagulation factor VIIa, coagulation factor VIII, coagulation factor IX, follicle stimulating hormone (FSH), anti-thrombin III, erythropoietin, granulocyte colony stimulating factor (GCSF), granulocyte macrophage colony stimulating factor (GMCSF), insulin, glucagon-like peptide-1 (GLP-1), agalsidase beta, alpha-glucosidase, laronidase, sphingomyelinase, phenylalanine hydroxylase, interleukin 2 (IL-2), interleukin 11 (IL-11), interleukin 12 (IL-12), or vascular endothelial growth factor (VEGF).

In some examples, the engineered protein is a hormone, an enzyme, or a cytokine.

In some examples, the hormone is insulin or an insulin analog.

In some examples, the multiple cells are beta cells, and the therapeutic agent is insulin.

In some examples, the container is a first container that contains the multiple cells, wherein the implantable device further includes one or more additional containers associated with the first container, and wherein each of the one or more additional containers contains additional, multiple cells.

In some examples, the first container and the one or more containers are spaced apart from one another.

In some examples, a flexible structure that maintains the first container and the one or more containers in a spaced configuration.

In some examples, the first container and the one or more additional containers are arranged in a matrix configuration.

In some examples, the implantable device is implanted into an arm or an abdomen of the subject.

In some examples, a body temperature of the subject at a time of implantation is between about 36 degrees Celsius and about 41 degrees Celsius.

In some examples, oxygen is delivered at a sustained and controlled rate for between about 1 year to about 6 years.

In some examples, oxygen is delivered at a sustained and controlled rate for about 5 years.

In some examples, the subject has type 1 diabetes.

In some examples, the subject has hemophilia.

In some examples, the subject has a growth hormone deficiency, Fabry disease, Pompe disease, mucopolysaccharidosis type I, Niemann-Pick disease type A, Niemann-Pick disease type B, or phenylketonuria.

In a seventeenth aspect, a not claimed method of treating a disease in a subject includes applying an accessory device against an exterior surface of a skin region of a subject in which an implantable device has been subcutaneously implanted along the skin region to promote delivery of oxygen to multiple cells contained within an interior region of the implantable device, thereby enhancing the delivery of oxygen to the multiple cells.

Examples may include one or more of the following features.

In some examples, the accessory device includes a transdermal patch that is configured to be secured to an exterior surface of the skin region, the transdermal patch including multiple microneedles that carry a substance capable of reacting to generate oxygen for the multiple cells within the interior region.

In some examples, the substance is hydrogen peroxide, carbamide peroxide, magnesium peroxide, or calcium peroxide.

In some examples, the substance is magnesium peroxide.

In some examples, a generation rate of oxygen is about 130 nL per minute per gram of magnesium peroxide.

In some examples, a volume of oxygen released about 30 µL per day.

In some examples, the generation of oxygen results in a flow rate of about 20 nL per minute.

In some examples, the accessory device includes a cuff that is configured to be secured to and to apply pressure to the skin region to direct a flow of blood towards the implantable device.

In some examples, the accessory device includes a sintered mesh that surrounds the implantable device and that carries a substance capable of reacting to generate oxygen.

In some examples, the multiple cells are human cells.

In some examples, the multiple cells are human beta cells.

In some examples, the therapeutic agent is an antibody or an antibody fragment, an enzyme, an engineered protein, or a protein fragment.

In some examples, the engineered protein is coagulation factor VIIa, coagulation factor VIII, coagulation factor IX, follicle stimulating hormone (FSH), anti-thrombin III, erythropoietin, granulocyte colony stimulating factor (GCSF), granulocyte macrophage colony stimulating factor (GMCSF), insulin, glucagon-like peptide-1 (GLP-1), agalsidase beta, alpha-glucosidase, laronidase, sphingomyelinase, phenylalanine hydroxylase, interleukin 2 (IL-2), interleukin 11 (IL-11), interleukin 12 (IL-12), or vascular endothelial growth factor (VEGF).

In some examples, the engineered protein is a hormone, an enzyme, or a cytokine.

In some examples, the hormone is insulin or an insulin analog.

In some examples, the multiple cells are beta cells, and the therapeutic agent is insulin.

In some examples, the implantable device includes a container defining the interior region and being configured to contain the multiple cells within the interior region. The container further defining first pores defined by an interior wall portion of the container, the first pores having a first size that (i) allows passage of the therapeutic agent and oxygen through the first pores and (ii) prevents passage of immune cells through the first pores. The container further defines second pores defined by an exterior wall portion of the container, the second pores having a second average size that is larger than the first average size, and the second pores being sized to promote vascularization and oxygen transfer along the exterior wall portion.

In some examples, the container is a first container that contains the multiple cells, wherein the implantable device further includes one or more additional containers associated with the first container, and wherein each of the one or more additional containers contains additional, multiple cells.

In some examples, the first container and the one or more additional containers are spaced apart from one another.

In some examples, the implantable device further includes a flexible structure that maintains the first container and the one or more additional containers in a spaced configuration.

In some examples, the first container and the one or more additional containers are arranged in a matrix configuration.

In some examples, the implantable device is implanted into an arm or an abdomen of the subject.

In some examples, a body temperature of the subject at the time of implantation is between about 36 degrees Celsius and about 41 degrees Celsius.

In some examples, oxygen is delivered at a sustained and controlled rate for between about 1 year to about 6 years.

In some examples, oxygen is delivered at a sustained and controlled rate for about 5 years.

In a not claimed comparative example, the interior region can be wide enough to accommodate multiple of the cells across a plane that is perpendicular to a central axis of the container.

In an eighteenth aspect, according to the present invention, an implantable device for providing a therapeutic agent for use in the treatment of a disease includes a container configured to contain multiple cells capable of producing the therapeutic agent within an interior region of the container. The container defines first pores defined by an interior wall portion of the container, the first pores having a first average size that (i) allows passage of the therapeutic agent and oxygen through the first pores and (ii) prevents passage of immune cells through the first pores. The container further defines second pores having a second average size that is larger than the first average size, the second pores being sized to promote vascularization and oxygen transfer along the exterior wall portion. The implantable device is to be administered to a patient in need thereof.

Embodiments may include one or more of the following features.

In some embodiments, the second average size of the second pores locates vasculature along the second pores, and wherein the second average size of the second pores allows passage of the therapeutic agent through the exterior wall portion to the vasculature.

In some embodiments, the first pores have first widths in a range of about 10 nm to 400 nm, wherein the second pores have second widths in a range of about 2 µm to about 60 µm, wherein the container has a wall thickness in a range of about 1 µm to about 100 µm, and wherein the implantable device has an external surface area to volume ratio of about 200 to about 5,000.

According to the present invention, the interior region has a width that is limited to accommodating a single cell of the multiple cells. The width may be in a range of 100 µm to 2 mm. In a not claimed comparative example, the interior region can be wide enough to accommodate multiple of the cells across a plane that is perpendicular to a central axis of the container.

In some embodiments, the container includes a tube, wherein, in a not claimed comparative example, the tube has a linear configuration, and wherein, according to the present invention, the tube has a spiral configuration or a helical configuration.

In some embodiments, the implantable device further includes a second tube disposed inside of the first tube, and wherein the first and second tubes together define an annular lumen for containing the multiple cells.

In some embodiments, the exterior wall portion is coated with a growth factor that promotes vascularization.

In some embodiments, the exterior wall portion is coated with a growth factor that promotes vascularization, and: wherein the growth factor is covalently linked to the exterior wall portion, and/or wherein the growth factor is electrostatically bound to the exterior wall portion, and/or wherein the growth factor is site-specifically bound to the exterior wall portion, and/or wherein the growth factor is selected from the group consisting of: vascular endothelial growth factor (VEGF), placenta derived growth factor (PDGF), transforming growth factor beta (TGFβ), and fibroblast growth factor (FGF), and/or wherein the growth factor is vascular endothelial growth factor (VEGF), placenta derived growth factor (PDGF), transforming growth factor beta (TGFβ), fibroblast growth factor (FGF), or a combination thereof, and/or wherein the growth factor is vascular growth factor (VEGF).

In some embodiments, the implantable device further includes the multiple cells contained within the interior region of the container.

In some embodiments, the multiple cells are beta cells, and the therapeutic agent is insulin.

In some embodiments, the implantable device further includes one or more additional containers associated with the container, wherein each of the one or more additional containers contains additional, multiple cells, and: wherein the container and the one or more additional containers are spaced apart from one another, or wherein the container and the one or more additional containers are coupled to each other in a matrix configuration.

In some embodiments, the patient has type 1 diabetes, hemophilia, or a growth hormone deficiency.

In some embodiments, the container includes a tubular wall that includes the interior and exterior wall portions, wherein the tubular wall further defines third pores arranged radially between the first and second pores, wherein the third pores have a third average size that is greater than the first average size and less than the second average size, wherein pores defined by the tubular wall gradually increase in size from an interior surface of the tubular wall to an exterior surface of the tubular wall, and wherein the container includes one or more of polycaprolactone, PTFE, ePTFE, nylon, polyether-ketone, polyether sulfone, polyester, polyvinylidene difluoride, and polysiloxane.

In some embodiments, the interior wall portion includes a tubular member and the exterior wall portion includes a coating that surrounds the tubular member, wherein the tubular member has a first material formulation and the coating has a second material formulation that is different from the first material formulation, wherein the first material formulation includes one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups, and wherein the second material formulation includes one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In a nineteenth aspect, according to the present invention, a therapeutic system for use in the treatment of a disease includes the implantable device of the eighteenth aspect, the implantable device configured to be implanted subcutaneously along a skin region. The therapeutic system further includes an accessory device configured to cooperate with the implantable device for promoting delivery of oxygen to the multiple cells. The therapeutic system is to be administered to a patient in need thereof.

Embodiments may provide one or more of the following advantages.

In some embodiments, the implantable device has a relatively large aspect ratio, which facilitates placement of the implantable device within narrow spaces at suitable locations within the body.

In some embodiments, the container of the implantable device is a flexible structure that facilitates positioning of the implantable device within asymmetric spaces of the body.

In some embodiments, the container of the implantable device is formed to impart a single-layer cellular arrangement that ensures that each cell can receive a sufficient amount of oxygen from passive diffusion of the oxygen through pores of the container alone and without an additional energy source for providing supplemental oxygen to the cells.

According to the present invention, a width of the interior region of the container is limited to accommodating only a single cell to impose this arrangement. This arrangement avoids the need for a supplemental source of oxygen and thereby avoids challenges that may otherwise be associated with such a supplemental source.

In some embodiments, a tubular shape and a size of the container also maximizes a ratio of external surface area to volume of the implantable device for providing the functional capability of passive oxygenation for the cells.

In some embodiments, the implantable device may be equipped with a tubular wall that is internal to a container and that, along with the container, forms an annular lumen in which the cells are arranged in a circumferential cellular layer that is imposed by an outer diameter of the internal tube. An arrangement of the cells along the circumferential cellular layer advantageously ensures that at least a portion of the surface area of each cell has direct access to pores of the container without any intervening cells. Such direct access ensures that each cell can receive a sufficient amount of oxygen from passive diffusion of the oxygen through the pores of the exterior and interior container alone and without an additional energy source for providing supplemental oxygen to the cells. The cellular arrangement allows the implantable device to have a minimal size, even while ensuring adequate oxygenation of the cells.

In some embodiments, the implantable device may be provided with an accessory device that can promote the delivery of oxygen to a local tissue that lies adjacent to the implantable device. For example, a cooperating accessory device (e.g., a cuff or a transdermal patch) may be placed against an exterior skin surface of the patient in the vicinity of implantable device to increase the concentration of oxygen in the local tissue to enhance the flow of oxygen during a short-term vascularization period following implantation.

In comparative examples, the implantable device may additionally include an active feature for providing supplemental oxygen to the cells without including an electromechanical energy source for powering the supply of oxygen, and thus avoiding challenges that may otherwise be associated with including such a source. For example, in some embodiments, the implantable device is loaded with a substance (e.g., a chemical substance) that can react to generate oxygen for increasing a supply of oxygen to the cells.

In some embodiments, the substance is encapsulated within particles that are sprayed onto an interior surface of the container of the implantable device or that are deposited within a dedicated reservoir of the container.

In some embodiments, the container of the implantable device has a double-layer wall construction that permits formation of pores within discrete size ranges.

In other embodiments, the container of the implantable device has a single-layer wall construction that may be manufactured in a simpler process.

Other aspects, features, and advantages will be apparent from the description, the drawings, and the claims.

### Description of Drawings

FIG. 1 is a side view of an implantable device according to a not claimed comparative example with a double-layer wall construction, a linear configuration, and a cellular arrangement for relying on passive oxygenation.
FIG. 2 is a side cross-sectional view of the implantable device of FIG. 1.
FIG. 3 is an enlarged side cross-sectional view of a portion of the implantable device of FIG. 1.
FIG. 4 is a top view of the implantable device of FIG. 1 according to an embodiment of the present invention formed into a spiral configuration.
FIG. 5 is a side view of the implantable device of FIG. 1 formed into the spiral configuration.
FIG. 6 is a side view of the implantable device of FIG. 1 according to another embodiment of the present invention formed into a helical configuration.
FIG. 7 is a top view of an implantable system according to a not claimed comparative example including multiple implantable devices of FIG. 1 provided in a parallel arrangement.
FIG. 8 is a top view of a portion of an implantable system according to a not claimed comparative example including multiple implantable devices of FIG. 1 provided in a matrix arrangement.
FIG. 9 is a side cross-sectional view of an implantable device according to a not claimed comparative example with a double-layer wall construction, a coaxial tube configuration, and a cellular arrangement for relying on passive oxygenation.
FIG. 10 is a side view of the implantable device of FIG. 9.
FIG. 11 is a side cross-sectional view of an implantable device according to a not claimed comparative example with a double-layer wall construction, a linear configuration, and a substance that provides for active oxygenation.
FIG. 12 is a side cross-sectional view of an implantable device according to a not claimed comparative example with a double-layer wall construction, a coaxial tube configuration, and a substance that provides for active oxygenation.
FIG. 13 is a side cross-sectional view of the implantable device of FIG. 12.
FIG. 14 is a side cross-sectional view of an implantable device according to a not claimed comparative example with a double-layer wall construction, a coaxial meshed tube configuration, and a substance that provides for active oxygenation.
FIG. 15 is a side view of an implantable device according to a not claimed comparative example that includes an outer meshed sleeve that is loaded with a substance that provides for active oxygenation.
FIG. 16 is a side view of a therapeutic system including an implantable device and a cuff.
FIG. 17 is a side view of a therapeutic system including an implantable device and a transdermal patch.
FIG. 18 is a schematic illustration of a manufacturing process including extrusion and electrospinning steps for producing the implantable devices of FIGS. 1, 9, 11, 13, 14, and 15.
FIG. 19 is a side cross-sectional view of a container of an implantable device produced according to the manufacturing process of FIG. 18.
FIG. 20 is a side view of an implantable device according to a not claimed comparative example with a single-layer wall construction, a linear configuration, and a cellular arrangement that relies on passive oxygenation.
FIG. 21 is a side cross-sectional view of the implantable device of FIG. 20.
FIG. 22 is an enlarged side cross-sectional view of a portion of the implantable device of FIG. 20.
FIG. 23 is a side cross-sectional view of an implantable device according to a not claimed comparative example with a single-layer wall construction, a coaxial tube configuration, and a cellular arrangement for relying on passive oxygenation.
FIG. 24 is a side cross-sectional view of an implantable device according to a not claimed comparative example with a single-layer wall construction, a linear configuration, and a substance that provides for active oxygenation.
FIG. 25 is a side cross-sectional view of an implantable device according to a not claimed comparative example with a single-layer wall construction, a coaxial tube configuration, and a substance that provides for active oxygenation.
FIG. 26 is a side cross-sectional view of an implantable device according to a not claimed comparative example with a single-layer wall construction, a coaxial meshed tube configuration, and a substance that provides for active oxygenation.
FIG. 27 is a side cross-sectional view of an implantable device according to a not claimed comparative example with a double-layer wall construction, a linear configuration, and a cellular arrangement for relying on passive oxygenation.
FIG. 28 is a side cross-sectional view of an implantable device according to a not claimed comparative example with a single-layer wall construction, a linear configuration, and a cellular arrangement for relying on active oxygenation.
FIG. 29 is a side cross-sectional view of an implantable device according to a not claimed comparative example with a double-layer wall construction, a linear configuration, and a single-layer circumferential cellular arrangement for relying on passive oxygenation.

### Detailed Description

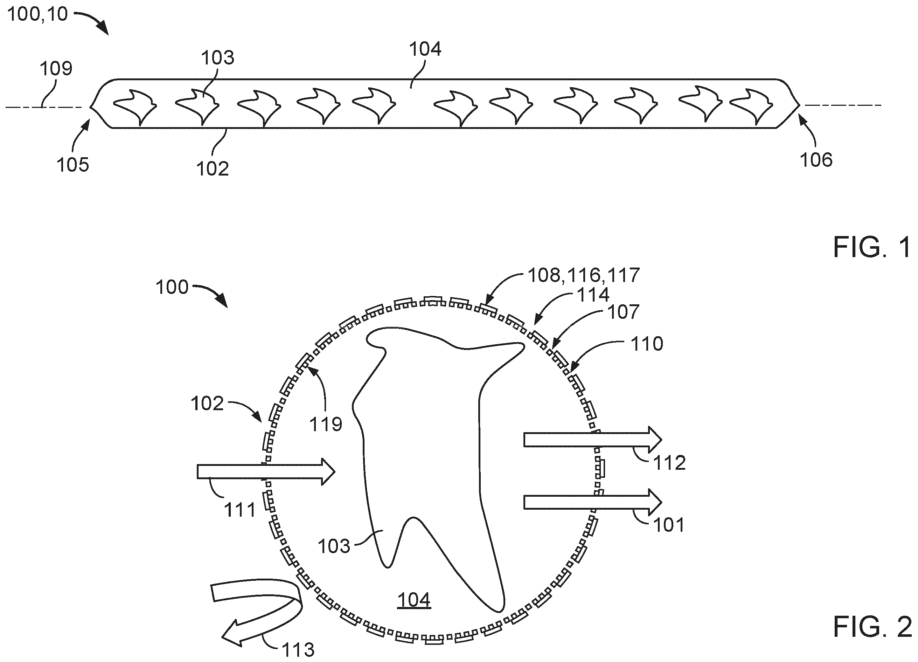

FIGS. 1 and 2 illustrate an implantable device 100 according to a comparative example that provides a therapeutic agent for treating a disease within the human body. For example, the implantable device 100 is designed to be implanted in a patient's body subcutaneously for providing insulin 101 to regulate the patient's blood glucose level for treating diabetes (e.g. type 1 or type 2 diabetes). In other examples, the implantable device 100 can be implanted to treat hemophilia. The implantable device 100 has a relatively large aspect ratio (e.g., the implantable device is relatively long and thin), which facilitates placement of the implantable device 100 within narrow spaces at suitable locations within the body. In some embodiments, the implantable device 100 has a surface area to volume ratio of about 200 to about 5000. Example sites in the body at which the implantable device 100 may be implanted include a patient's forearm, abdomen, back of the arm, or lower back. Techniques that may be used to implant the implantable device 100 include incision and endoscope, depending on the site of implantation. The implantable device can be implanted in a patient when the body temperature of the patient is between about 36 degrees and 41 degrees Celsius (e.g., about 37 degrees Celsius).

The implantable device 100 can typically perform its therapeutic function within the body for a period of about 24 months to about 36 months, and in some cases, even up to a period of about 60 months. Once the implantable device 100 loses its capability to perform its therapeutic function, the implantable device 100 may be removed and optionally replaced.

The implantable device 100 includes a container 102 and multiple cells 103 that are located within an interior region 104 of the container 102. In this regard, the interior region 104 forms a cell reservoir. The cells 103 are human beta cells that, under the appropriate conditions, are capable of producing and secreting insulin 101 that is then released from the container 102 to surrounding vasculature 115. The implantable device 100 is therefore designed to provide beta cell replacement therapy (BCRT).

The container 102 has a generally tubular shape that provides a linear configuration 10 of the implantable device 100. The container 102 is closed (e.g., sealed) at opposite ends 105, 106 to maintain the cells 103 within the interior region 104. The container 102 includes an interior wall 107 and an exterior wall 108 that are adhered to each other and that are concentrically arranged with respect to a central axis 109 of the container 102.

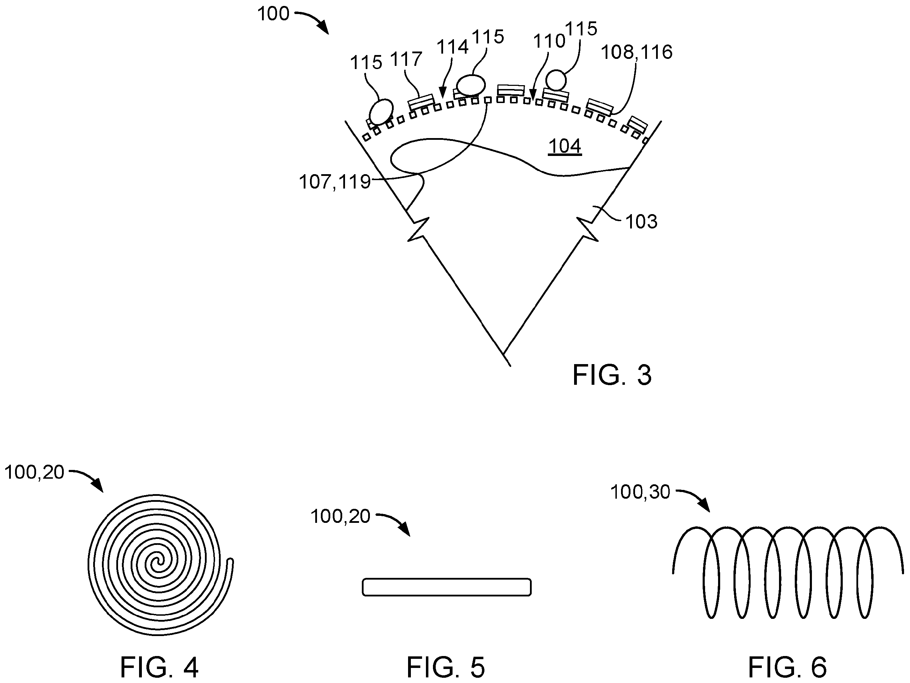

Referring to FIG. 3, features of the interior and exterior walls 107, 108 of the container 102 determine significant functional capabilities of the implantable device 100. For example, the interior wall 107 defines pores 110 (e.g., through openings) that are sized to allow passage of nutrients 111 needed by the cells 103 to remain viable and to produce insulin 101. Such nutrients 111 that are permitted to diffuse through the pores 110 into the interior region 104 of the container 102 include water, oxygen, glucose, and various other nutrients. The pores 110 are also sized to allow passage of insulin 101 and waste products 112 (e.g., carbon dioxide) produced by the cells 103. Accordingly, the nutrients 111 pass into the container 102, while the insulin 101 and the waste products 112 pass out of the container 102.

The pores 110 are also sized to prevent passage of the cells 103 out of the interior wall 107. Therefore, along with closure of the ends 105, 106 of the container 102, the pores 110 are responsible for containing the cells 103 within the container 102. The cells 103 are recognizable by components 113 (e.g., antibodies and immune cells) of the patient's immune system as foreign bodies. Importantly, however, the pores 110 are also sized to prevent passage of these components 113 into the container 102 to isolate the cells 103 from the components 113, thereby protecting the cells 103 from attack by the components 113. Accordingly, the pores 110 of the interior wall 107 provide the implantable device 100 with the functional capability of immunoisolation for protecting the cells 103 from the immune system. The pores 110 may have a circular or a non-circular cross-sectional shape. The pores 110 typically have widths in a range of about 10 nm to about 400 nm (e.g., about 40 nm). Techniques that may be used to measure or confirm the sizes of the pores 110 include bubble point techniques (e.g., where the pressure required to force an air bubble through a pore is inversely proportional to the size of the pore), diffusion and image analysis techniques that utilize a difference between a total area and a projected area, and Brunauer-Emmett-Teller (BET) techniques, such as mercury porosimetry and scanning electron microscopy. The pores 110 can provide the above-mentioned functionalities across this full range of widths. In contrast, in some instances, the above-mentioned components 113 of the immune system may have widths that are at least as large as about 2 µm, and the cells 103 typically have widths that are at least as large as about 100 µm. In some examples, the geometry of the membrane may influence the behavior of inflammatory cells due to the complexity of the immuno-response reaction such that behavior of the immune components 113 at the implantable device 100 may be affected by more than just pore size alone.

The exterior wall 108 defines pores 114 (e.g., through openings) that are in fluid communication with the pores 110 of the interior wall 107. The pores 114 are larger than the pores 110 such that the pores 114 permit passage of the nutrients 111, the insulin 101, and the waste products 112. The pores 114 also allow passage of the components 113 of the immune system. However, these components 113 are ultimately blocked from entering into the interior region 104 of the container 102 by the pores 110 of the interior wall 107.

Importantly, the pores 114 are sized to promote vascularization of the implantable device 100 and therefore to promote oxygen transfer along the exterior wall 108. That is, the larger size of the pores 114 allows nearby blood vessels 115 (e.g., capillaries), carrying oxygen and the nutrients 111, to position themselves adjacent the pores 114 (e.g., within the pores 114 or in contact with the exterior wall 108 and extending across the pores 114). The blood vessels 115 may include preexisting blood vessels or new blood vessels that have sprouted from preexisting blood vessels that are located within a certain vicinity of the implantable device 100 (e.g., within a distance of about 15 µm from the implantable device 100). In some instances, the larger size of the pores 114 may encourage the growth of new blood vessels towards the implantable device 100 (e.g., via chemical and/or mechanical signaling). In some instances, the larger size of the pores 114 allows for positioning of blood vessels within the pores 114. In general, a pore size that is too large may result in the presence of connective or fibrous tissue near the pores, whereas a pore size that is too small may block vascularization of the implantable device 100. Accordingly, the pores 114 of the exterior wall 108 provide the container 102 with the functional capability of promoting vascularization along an exterior surface 116 of the container 102.

The pores 114 may have a circular or a non-circular cross-sectional shape. The pores 114 typically have widths in a range of about 2 µm to about 60 µm (e.g., about 5 µm). The sizes of the pores 114 may be measured or confirmed using the techniques described above with respect to the pores 110. The blood vessels 115 typically have widths (e.g., diameters) in a range of about 5 µm to about 50 µm.

Additionally, the container 102 may also include a coating of one or more growth factors 117 across the exterior surface 116 for further promoting vascularization. Example growth factors 117 that may be coated across the exterior wall 108 of the container 102 include vascular endothelial growth factor (VEGF), fibroblast growth factor (FGF), transforming growth factor beta (TGF-β), and platelet-derived growth factor (PDGF). Alternatively or additionally, other agents that indirectly promote vascularization may be applied to the exterior surface 116 of the container 102, such as angiopoetin-1 or bone morphogenic protein-2 (BMP-2). The growth factors 117 may be covalently linked across the exterior wall 108 of the container 102, electrostatically bound to the exterior wall 108 of the container 102, or site-specifically bound to the exterior wall 108 of the container 102. The site specific linkage via thiol-maleimide or thiol-vinyl sulfone addition reaction can be achieved involving thiol groups of cysteine amino acids in the proteins and maleimide or vinyl sulfone groups introduced to the implantable device surface. In addition, by introducing thiol groups to device surface, the vascularization promoting proteins can be linked through disulfide bond using protein's cysteine residue. Other methods of site specific conjugation involves oxime bond between carbohydrate residues in the proteins (particularly glycoproteins) and aminooxy groups on the implantable device surface. Furthermore, by incorporating unnatural amino acids containing specific functional groups as azide groups to the growth factor proteins either by protein engineering or chemical modifications, these proteins can also be linked to the device via click chemistry after introducing alkyne groups to the device surface. As a result of either or both of the sizes of the pores 114 and the presence of one or more growth factors 117 or other agents, the implantable device 100 is typically vascularized to an extent that the blood vessels 115 can continually provide adequate concentrations of oxygen and the nutrients 111 within a period of about two weeks to about three weeks following implantation. Blood flowing through the blood vessels 115 delivers the nutrients 111 to the implantable device 100 and takes up the insulin 101 and the waste products 112 secreted by the cells 103 within the interior region 104 of the container 102.

The cells 103 are present within the interior region 104 of the container 102 in an amount that is sufficient to produce a therapeutic effect (e.g., to produce an adequate amount of insulin 101 to treat diabetes on an ongoing basis). For example, the interior region 104 of the container 102 typically has a volume capacity of about 0.1 mL to about 1.5 mL.

Moreover, the cells 103 are arranged within the container 102 in a manner such that each of the cells 103 can receive a sufficient amount of oxygen from passive diffusion of the oxygen through the pores 114, 110 of the exterior and interior walls 108, 107 alone and without an additional energy source for providing supplemental oxygen to the cells 103. Were the cells 103 to be arranged in multiple, adjacent cell layers within the interior region 104 of the container 102, oxygen diffusing passively through the pores 110 would not reach the cells 103 located most interiorly in an amount that would be sufficient for maintaining the cells 103 in a viable state, causing these interior cells 103 to die. Therefore, a width (e.g., a diameter) of the interior region 104 of the container 102 is limited to accommodating a single cell 103 along the central axis 109 of the container 102, as shown in FIG. 1. Accordingly, increasing a concentration of the cells 103 within the interior region 104 decreases an average axial spacing between adjacent cells 103. In some embodiments, adjacent cells 103 may be spaced apart within the interior region 104 by an axial distance of up to about 300 µm. In some embodiments, adjacent cells 103 may be in contact with each other (e.g., with no spacing between cells 103).

Such an arrangement that provides the single cell layer within the container 102 ensures that oxygen diffusing passively through the pores 110 can reach each of the cells 103 at a concentration that is sufficient for survival of the cells 103, while allowing the implantable device 100 to have a minimal size. Accordingly, the interior region 104 of the container 102 typically has a width in a range of about 100 µm to about 2 mm. The approximate single-cell width of the interior region 104 ensures that at least a portion of the surface area of each cell 103 (e.g., provided by the cell membrane) has direct exposure to pores 110 of the interior wall 107 (e.g., without any intervening cells 103). Thus, this arrangement avoids the need for a supplemental source of oxygen and thereby avoids challenges that may otherwise be associated with such a supplemental source, such as over-oxygenating cells contained within an implant, undesirably increasing a size of the implant to accommodate an energy source (e.g., an electromechanical energy source) for producing the oxygen, undesirably increasing a complexity of the implant to accommodate the energy source, and undesirably increasing a degree of user interaction with the implant (e.g., to charge a battery that serves as an energy source for powering the production of oxygen).

In part due to the small width of the interior region 104 of the container 102, the implantable device 100 has a relatively low, narrow profile that facilitates fitting of the implantable device 100 at a selected location within the body. The tubular shape and the size of the container 102 also maximize a ratio of external surface area to volume of the implantable device 100 for providing the functional capability of passive oxygenation for the cells 103. For example, the container 102 has an external surface area to volume ratio that is typically between about 1,000 and about 10,000. The container 102 typically has a wall thickness in a range of about 1 µm to about 100 µm such that the implantable device 100 has a total width in a range about 0.1 mm to about 2 mm. Individually, the interior wall 107 typically has a wall thickness in a range of about 5 µm to about 25 µm, and the exterior wall 108 typically has a wall thickness in a range of about 20 µm to about 150 µm. The implantable device 100 typically has a length in a range about 1 m to about 60 m. Accordingly, the implantable device 100 is embodied as a long, thin tube.

Furthermore, the container 102 of the implantable device 100 is a flexible structure, and the flexibility of the container 102 facilitates positioning of the implantable device 100 within asymmetric spaces of the body.

In some embodiments, the interior wall 107 of the container 102 may be formed as a porous tube from an extrusion process. For example, in some embodiments, the interior wall 107 may be extruded from one or more hollow fiber membrane (HFM) materials, such as expanded polytetrafluoroethylene (ePTFE), mixed cellulose ester, polyethersulfone (PES), and modified PES. In other embodiments, the interior wall 107 may be extruded from alginate that is cross-linked with divalent cations. In other embodiments, the interior wall 107 may be extruded from one or more of polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups.

In some embodiments, the exterior wall 108 of the container 102 may be formed as a porous coating from an electrospinning process. For example, in some embodiments, the exterior wall 108 may be electrospun from one or more fibrous materials, such as polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

In some embodiments, the implantable device 100 may be adjusted (e.g., bent, biased, or otherwise deformed) from the linear configuration 10 shown in FIG. 1 into a round, curved, or otherwise non-linear configuration. For example, FIGS. 4 and 5 illustrate an embodiment according to the present invention of the implantable device 100 formed into a spiral configuration 20. In the spiral configuration 20, the implantable device 100 has a length that is reduced relative to that of the implantable device 100 in the linear configuration 10 and that falls in a range of about 25 mm to about 225 mm. In some embodiments, the length may be influenced by one or more parameters, such as how many cells a person needs, diameters of the cells, packing in the tube, and the diameter of the tube. The reduced length advantageously allows implantation of the implantable device 100 within relatively short spaces within the body. The spiral configuration 20 may have additional advantages as compared to the linear configuration 10. For example, one relatively large spiral can help to simplify manufacturing since only one long, thin tube may need to be filled and sealed. Accordingly, only one spiral-shaped device may need to be implanted instead of multiple long, linear implants. In some embodiments, multiple spiral-shaped devices may be implanted in a stacked configuration, which may help to minimize an overall footprint. The implantable device 100 has an overall width in the spiral configuration 20 (e.g., equal to the length of about 25 mm to about 225 mm) that is increased relative to that of the implantable device 100 in the linear configuration 10. The implantable device 100 may be maintained in the spiral configuration 20 by a meshed or molded support structure to which the implantable device 100 may be attached.

FIG. 6 illustrates another embodiment according to the present invention of the implantable device 100 formed into a helical configuration 30. In the helical configuration 30, the implantable device 100 has a length that is reduced relative to that of the implantable device 100 in the linear configuration 10 and that falls in a range of about 15 mm to about 500 mm. The implantable device 100 has an overall width in the helical configuration 30 that is increased relative to that of the implantable device 100 in the linear configuration 10 and that falls in a range of about 2 mm to about 35 mm. The helical configuration 30 may have advantages as compared to the linear configuration 10. For example, one relatively large helix can help to simplify manufacturing since only one long, thin tube may need to be filled and sealed.

Accordingly, only one helical device may need to be implanted instead of multiple long, linear implants. The implantable device 100 may be maintained in the helical configuration 30 by a meshed or molded support structure to which the implantable device 100 may be attached.

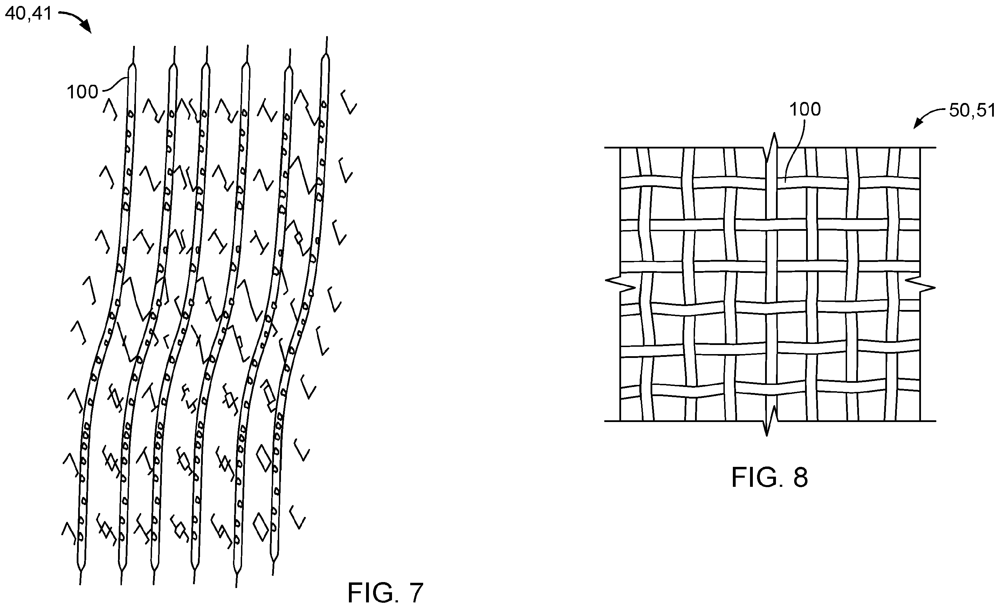

In some embodiments, multiple implantable devices 100 may be utilized together or coupled to one another to form an implantable system. For example, FIG. 7 illustrates an implantable system 40 according to a not claimed comparative example that includes multiple implantable devices 100 provided in the linear configuration 10. The implantable devices 100 are spaced apart from each other by a distance of about 0.2 mm to about 2 mm (e.g., about 1 mm) in a parallel arrangement 41 to ensure access to vascularization along the entire exterior surface 116 of each implantable device 100. In some embodiments, the implantable system 40 may include a total of 2 to 75 implantable devices 100, such that the implantable system 40 typically has a total width in a range of about 2 mm to about 100 mm.

In some embodiments, the implantable devices 100 may be interwoven into a matrix configuration (e.g., a fiber mesh) that maintains the implantable devices 100 in the spaced apart arrangement. Owing to the flexibility of the fiber mesh, the implantable devices 100 are afforded some play in position, even while being maintained apart from one another. In some embodiments of the implantable system 40, the implantable devices 100 may alternatively or additionally be dispersed within a scaffold of a gel.

FIG. 8 illustrates an implantable system 50 according to a not claimed comparative example that includes multiple implantable devices 100 provided in the linear configuration 10. In particular, the implantable devices 100 are provided at right angles to each other to form a matrix arrangement 51 in which adjacent implantable devices 100 are spaced apart from each other by a distance of about 0.2 mm to about 2 mm to ensure access to vascularization along the majority of the exterior surface 116 of each implantable device 100. In some embodiments, the implantable system 50 may include a total of 4 to 70 implantable devices 100, such that the implantable system 50 typically has a total width in a range of about 20 mm to about 100 mm and a total length in a range of about 100 mm to about 250 mm.

In some embodiments, an implantable system that is similar in construction and function to the implantable systems 40, 50 may include multiple implantable devices 100 provided in the spiral configuration 20 or in the helical configuration 30.

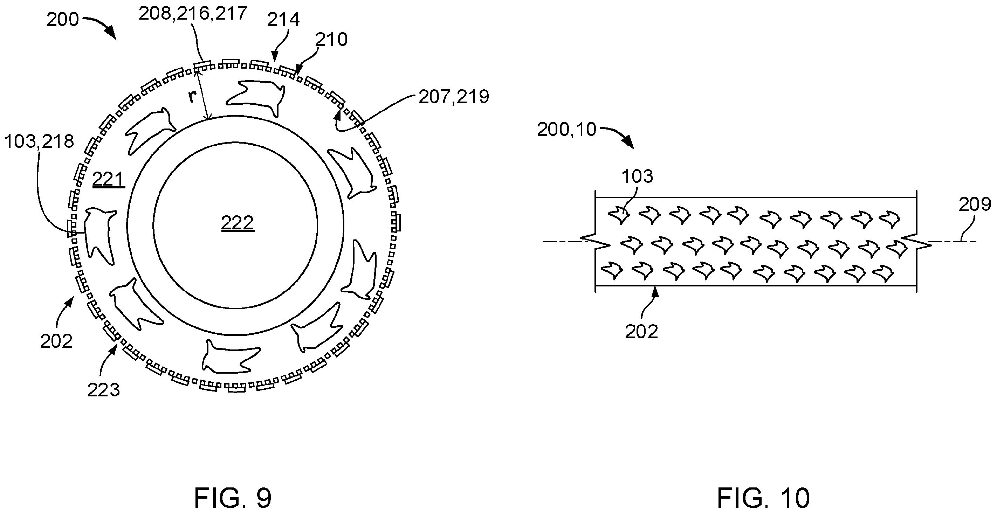

In some embodiments, an implantable device that is similar in construction and function to the implantable device 100 may be equipped with a tubular wall that is internal to a container. For example, FIGS. 9 and 10 illustrate such an implantable device 200 (pores not visible in figures). The implantable device 200 includes a container 202, an internal tube 220 that is arranged coaxially with the container 202 within an interior region of the container 202, and cells 103 that lie between the container 202 and the internal tube 220. The container 202 is substantially similar in construction and function to the container 102, except that the container 202 has a length and a width that are different from those of the container 102. Accordingly, in some embodiments, the implantable device 200 has an aspect ratio of about 100 to about 2,000. The container 202 includes an interior wall 207 that defines pores 210 and an exterior wall 208 that surrounds the interior wall 207 and defines pores 214. The pores 210, 214 are respectively sized equivalently to the pores 110, 114 of the walls 107, 108 of the container 102 to provide the functional capabilities discussed above with respect to the pores 110, 114. Like the container 102, the container 202 is closed (e.g., sealed) at opposite ends.

The container 202 and the internal tube 220 together form an annular lumen 221 in which the cells 103 are arranged in a circumferential cell layer 218 that is imposed by an outer diameter of the internal tube 220. Such an arrangement still enables the implantable device 200 to meet the ratio of external surface area (e.g., along the exterior surface 216) to volume of the annular lumen 221 of about 500. A radial length r of the annular lumen 221 is limited to accommodating a single cell 103 along an inner surface 219 of the container 202. Accordingly, the radial length r of the annular lumen 221 is in a range of about 0.5 mm to about 4 mm. An arrangement of the cells 103 along the circumferential cell layer 218 advantageously ensures that at least a portion of the surface area of each cell 103 has direct access to pores 210 of the interior wall 207 (e.g., without any intervening cells 103). Such direct access ensures that each cell 103 can receive a sufficient amount of oxygen from passive diffusion of the oxygen through the pores 214, 210 of the exterior and interior walls 208, 207 alone and without an additional energy source for providing supplemental oxygen to the cells 103.

The cells 103 are present within the annular lumen 221 of the implantable device 200 in an amount that is sufficient to produce a therapeutic effect. For example, the annular lumen 221 typically has a volume capacity in a range of about 0.1 mL to about 1.1 mL. In some embodiments, 4 to 100 cells 103 may be present around the annular lumen 221 at any cross-sectional position of the circumferential cell layer 218 (e.g., at any axial position along the central axis 209 of the container 202). Accordingly, increasing a concentration of the cells 103 within the annular lumen 221 decreases an average angular spacing between adjacent cells 103 at the same axial position.

A cell arrangement provided by the single cell layer within the container 202 ensures that oxygen diffusing passively through the pores 210 can reach each of the cells 103 at a concentration that is sufficient for survival of the cells 103, while allowing the implantable device 200 to have a minimal size. The radial length r of the annular lumen 221 avoids the need for a supplemental source of oxygen and thereby avoids the above-discussed challenges that may otherwise be associated with such a supplemental source. The total number of cells 103 within the container 202 is determined by the length and the outer diameters of the container 202 and the internal tube 220. The internal tube 220 defines a core region 222 that may be empty (e.g., void of cells, as shown in FIG. 9) or alternatively formed as a solid.

The container 202 and the internal tube 220 together form a flexible housing 223 of the implantable device 200. In some embodiments, the housing 223 provides mechanical stiffness that can resist collapse during use. In some embodiments, the interior and exterior walls 207, 208 have the same material formulations as those discussed above with respect to the interior and exterior walls 107, 108 of the container 102. In some embodiments, the internal tube 220 may be made of one or more hydrophobic materials that resist water (e.g., prevent absorption or flow through of water).

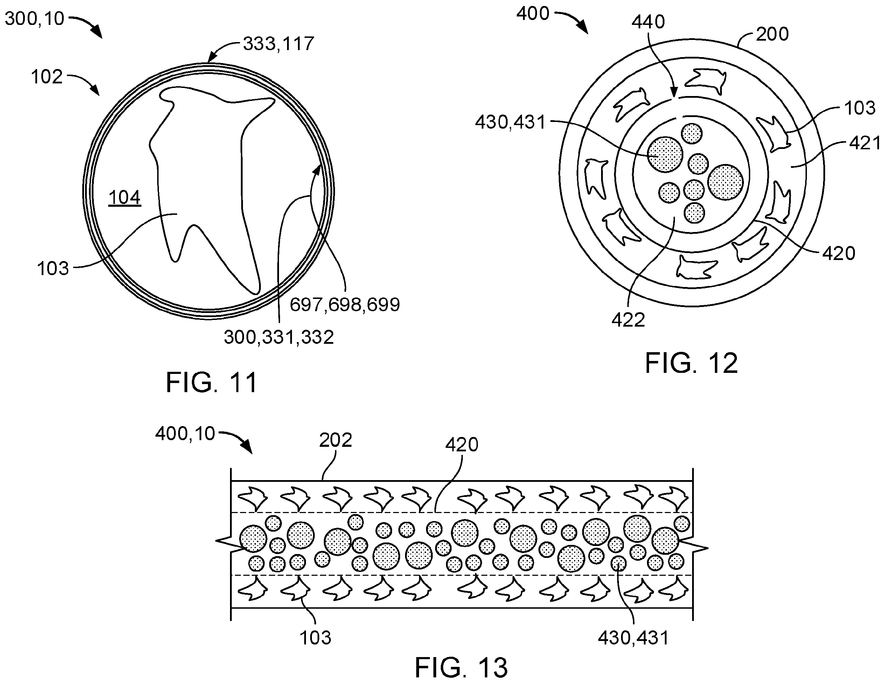

While the implantable devices 100, 200 have been described and illustrated as being designed to allow for oxygenation of the cells 103 by utilizing a passive transport process (e.g., diffusion) and without providing the cells 103 with supplemental oxygen, in some comparative examples, an implantable device that is similar in construction and function to either of the implantable devices 100, 200 may additionally include an active feature for providing supplemental oxygen to the cells 103. Such an implantable device may include this active feature without including an electromechanical energy source for powering the supply of oxygen, and thus avoiding the above-discussed challenges that may otherwise be associated with including such a source. For example, FIG. 11 illustrates an implantable device 300 (pore structure not visible in figure) that is loaded with a substance 330 (e.g., an oxygenation agent) that can react to actively generate oxygen (e.g., supplemental oxygen) at a controlled rate over a short-term period of about two weeks to about three weeks while the implantable device 300 is vascularized.

The implantable device 300 is otherwise substantially similar in construction and function to the implantable device 100. Accordingly, in addition to the substance 330, the implantable device 300 includes the container 102 and the cells 103. The substance 330 is encapsulated within particles 331 that are sprayed onto an inner surface 119 of the container 102 to form a thin coating 332. The implantable device 300 also includes a hydrophobic polymer 333 that is coated onto the exterior surface 116 of the container 102. During the vascularization period (e.g., when a concentration of oxygen delivered to the cells 103 via passive diffusion may be insufficient to maintain the cells 103 in a viable state or otherwise may be at a sub-optimal level), the substance 330 can react to generate and release oxygen directly to the cells 103 within the interior region 104 of the container 102.

Example substances 330 that may be encapsulated within the particles 331 include hydrogen peroxide, carbamide peroxide, magnesium peroxide, or calcium peroxide. Example hydrophobic polymers 333 coated onto the exterior surface 116 include a siloxane polymer or a polycaprolactone (PCL). For example, in some embodiments, when the substance 330 is magnesium peroxide (MgO2) or hydrogen peroxide (H2O2), oxygen can be generated at a rate within a suitable range of rates, such as at a rate of about 130 nL per minute per gram of substance 330. In some embodiments, the volumetric rate of oxygen released by the substance 330 falls within a suitable range of volumetric rates, such as at a volumetric rate of about 30 µL per day. In some embodiments, a coating may be applied to the implantable device to change the release rate. In some embodiments, the amount of oxygen released can be measured using a pressure sensor. In some embodiments, the substance 330 may be carbamide peroxide (CH6N2O3) or calcium peroxide (CaO2).

In another comparative example, FIGS. 12 and 13 (container pores not visible in figures) illustrates an implantable device 400 that is also loaded with a substance 430 for actively generating oxygen at a controlled rate over the short-term vascularization period of about two weeks to about three weeks. In addition to including the substance 430, the implantable device 400 is otherwise substantially similar in construction and function to the implantable device 200, except that the implantable device 400 includes an internal tube 420 instead of the internal tube 220. Accordingly, the implantable device 400 further includes the container 202 (e.g., surrounding the internal tube 420) and the cells 103. The substance 430 is encapsulated within particles 431 that are located within a core region 422 of the internal tube 420. During the vascularization period, the substance 430 can react to generate oxygen. The internal tube 420 defines pores 440 (e.g., through openings) that are sized to allow passage of oxygen. Therefore, the oxygen generated by the substance 430 is released through the pores 440 to the cells 103 within an annular lumen 421 of the implantable device 400.

Example substances 430 that may be encapsulated within the particles 431 include hydrogen peroxide, carbamide peroxide, magnesium peroxide, or calcium peroxide. Furthermore, the substance 430 may exhibit or result in any of the quantitative or qualitative parameters discussed above with respect to the example substances as described for the example substance 330.

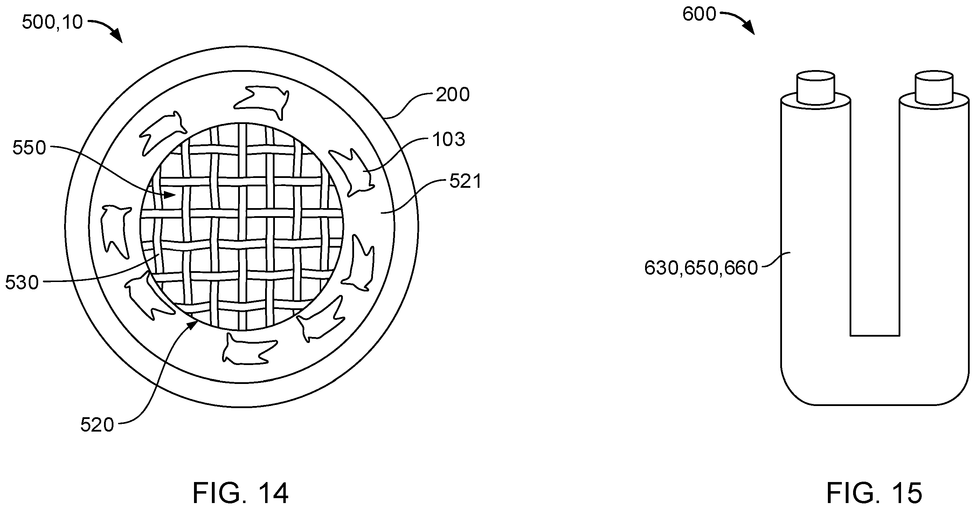

FIG. 14 also illustrates an implantable device 500 (container pores not visible in figure) according to a comparative example that is loaded with a substance 530 for actively generating oxygen at a controlled rate over the short-term vascularization period of about two weeks to about three weeks. The implantable device 500 is similar in construction and function to the implantable device 400, except that the implantable device 500 includes an internal tube 520 that is formed from a sintered matrix 550 instead of including the internal tube 420 with the core region 422 containing the particles 431. Accordingly, the implantable device 500 further includes the container 202 (e.g., surrounding the internal tube 520) and the cells 103. The substance 530 is loaded within the sintered matrix 550 of the internal tube 520. During the vascularization period, the substance 530 can react to generate oxygen. Peripheral edges of the sintered matrix 550 form outer openings that are large enough to allow the passage of oxygen. Therefore, oxygen generated by the substance 530 is released from the sintered matrix 550 directly to the cells 103 within an annular lumen 521 of the implantable device 500.

Example substances 530 that may be loaded within the sintered matrix 550 include hydrogen peroxide, carbamide peroxide, magnesium peroxide, or calcium peroxide. Furthermore, the substance 530 may exhibit or result in any of the quantitative or qualitative parameters discussed above with respect to the example substances as described for the example substance 330.

In some embodiments, an implantable device that is substantially similar in construction and function to any of the implantable devices 100, 200, 300, 400, 500 may be further equipped with an outer sleeve that is loaded with an oxygenation agent. For example, FIG. 15 illustrates an implantable device 600 according to a comparative example that includes such an outer sleeve 660 that is loaded with a substance 630 for actively generating oxygen at a controlled rate over the short-term vascularization period of about two weeks to about three weeks. The implantable device 600 is otherwise substantially similar in construction and function to any of the above-discussed implantable devices 100, 200, 300, 400, 500, such that the implantable device 600 further includes a housing 602 that is surrounded by the outer sleeve 660 and cells 103 that are located within the housing 602. The housing 602 may be embodied as any of the containers or arrangements 102, 202 and 220, 202 and 420, or 202 and 520. While the implantable device 600 is illustrated with a U shape of a relatively reduced length, in some comparative examples, the implantable device 600 may be provided in a straight (e.g., linear) configuration or in another configuration with a different shape.

The outer sleeve 660 is formed from a sintered matrix 650 that is loaded with the substance 630, which can react to generate oxygen during the vascularization period. Openings in the sintered matrix 650 along an inner surface of the outer sleeve 660 are large enough to allow the passage of oxygen. Therefore, oxygen generated by the substance 630 is released from the sintered matrix 650 around the housing 602 and can diffuse to the cells 103 through pores of the housing 602.

Example substances 630 that may be loaded within the sintered matrix 650 include hydrogen peroxide, carbamide peroxide, magnesium peroxide, or calcium peroxide. Furthermore, the substance 630 may exhibit or result in any of the quantitative or qualitative parameters discussed above with respect to the example substances as described for the example substance 330.

In some comparative examples, any of the implantable devices 300, 400, 500, 600 may be modified to accommodate a higher cell density and therefore include an active oxygenation configuration that is suitable for providing supplemental oxygen over a long-term period of about two years to about three years. For example, such an implantable device may be sized larger than any of the implantable devices 300, 400, 500, 600 to include an interior region or an annular lumen that is wide enough to accommodate more than one cell along an axial cross section of the interior region or annular lumen.

In some embodiments, any of the implantable devices 300, 400, 500, and 600 may further include additional particles 697 (e.g., stable nano- or micro-carriers) that encapsulate a catalyst 698 for modulating the rate of peroxide diffusion. The particles 697 may be applied to the inner surface 119, 219 of the interior wall 107, 207 of the implantable device 300, 400, 500, 600 as part of a coating 699, as illustrated in FIG. 11. For example, in some embodiments, the substance 330, 430, 530, 630 may be hydrogen peroxide, and the catalyst 698 may be catalase.

In a not claimed comparative example, any of the implantable devices 200, 300, 400, 500, 600 may be provided in the linear configuration 10, in embodiments according to the present invention, any of the implantable devices 200, 300, 400, 500, 600 is provided in any one of the spiral or helical configurations 20, 30 discussed above with respect to the implantable device 100. Furthermore, multiple implantable devices 100, 200, 300, 400, 500, 600 may be provided as part of an implantable system with a parallel arrangement 41 or a matrix arrangement 51, as discussed above with respect to the implantable systems 40, 50.

While the above-discussed implantable devices 200, 400, 500 have been described and illustrated with cylindrical-shaped internal tubes 220, 420, 520 that are concentrically arranged within an outer container, in some embodiments, an implantable device that is otherwise similar in construction and function to the any of the implantable devices 200, 400, 500 may alternatively include an elongate internal member that has a non-circular cross-sectional shape. In some embodiments, an implantable device that is otherwise similar in construction and function to the any of the implantable devices 200, 400, 500 may alternatively include an elongate internal member that is not concentrically arranged with respect to an outer container. For example, the elongate internal member may be positioned off-axis within a surrounding outer container.

In some embodiments, any of the implantable devices 100, 200, 300, 400, 500, 600 may be provided with an accessory device that can promote the delivery of oxygen to a local tissue that lies adjacent to the implantable device. For example, a therapeutic system may include an implantable device that is implanted subcutaneously and a cooperating accessory device that is placed against an exterior skin surface of the patient in the vicinity of implantable device to increase the concentration of oxygen in a local tissue surrounding the implantable device. The therapeutic system may be designed to enhance the flow of oxygen during a short-term vascularization period of about two weeks to about three weeks. In some examples, the patient may utilize the accessory device at regular intervals (e.g., once per week) to promote vascularization.

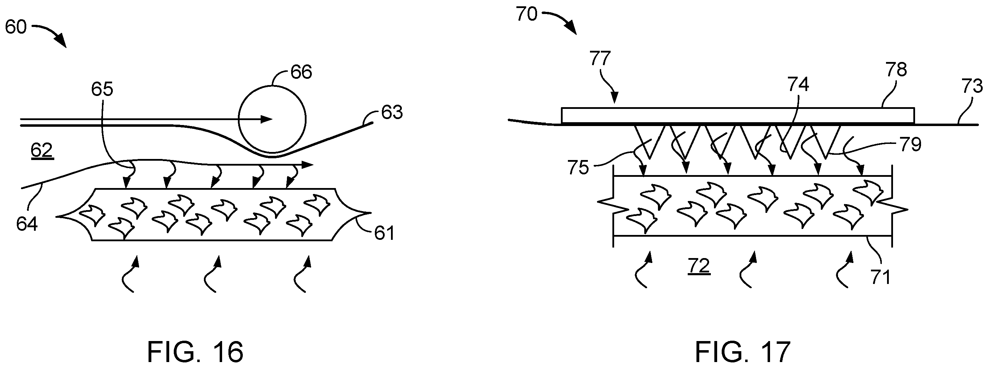

FIG. 16 illustrates one such therapeutic system 60. The therapeutic system 60 includes an implantable device 61 that is implanted subcutaneously within a tissue 62 beneath a skin layer 63 and an accessory device that is positioned adjacent an exterior surface of the skin layer 63. The implantable device 61 may be embodied as any of the implantable devices 100, 200, 300, 400, 500, 600. The accessory device is provided as a cuff 66 (e.g., such as a massage cuff) that can be placed around that patient's body adjacent the skin layer 63 to compress (e.g., squeeze) the skin layer 63 towards the implantable device 61 at regular time intervals. Pressure applied by the cuff 66 to the skin layer 63 directs a flow of blood 64 towards the implantable device 61 and increases a flow rate of blood 64 within the tissue 62. In this manner, the cuff 66 increases or otherwise enhances the flow of oxygen 65 to the tissue 62. A higher concentration of oxygen within the tissue 62 increases an overall amount of oxygen that is available to reach the cells 103 within the implantable device 61.

Another therapeutic system 70 is illustrated in FIG. 17. The therapeutic system 70 includes an implantable device 71 that is implanted subcutaneously within a tissue 72 beneath a skin layer 73 and an accessory device that is positioned adjacent an exterior surface of the skin layer 73. The implantable device 71 may be embodied as any of the implantable devices 100, 200, 300, 400, 500, 600. The accessory device is provided as a transdermal patch 77 that includes a base layer 78 and microneedles 79. The microneedles 79 extend from the base layer 79 and pierce the skin layer 73 to access the tissue 72. The microneedles 79 are loaded with a substance 74 (e.g., an oxygenation agent) that can react to generate oxygen 75. In some embodiments, the microneedles 79 may be made of one or more materials, such as metals, polymers including but not limited to PLGA, PCL, hyalrunic acid, etc. These polymer-based microneedles can be crosslinked with appropriate crosslinking agents to maintain their stability. In some embodiments, the rate of oxygen release is measured by the amount of dissolved oxygen using oxygen sensors (e.g., electromechanical or optical-based optical sensors).

Example substances 74 that may be loaded within the microneedles 79 include hydrogen peroxide, carbamide peroxide, magnesium peroxide, or calcium peroxide. Furthermore, the substance 74 may exhibit or result in any of the quantitative or qualitative parameters discussed above with respect to the example substances as described for the example substance 330.

The oxygen 75 is released from the microneedles 79 to the tissue 72 and thereby provides an overall increased amount of oxygen 75 that is available to reach the cells 103 within the implantable device 71.

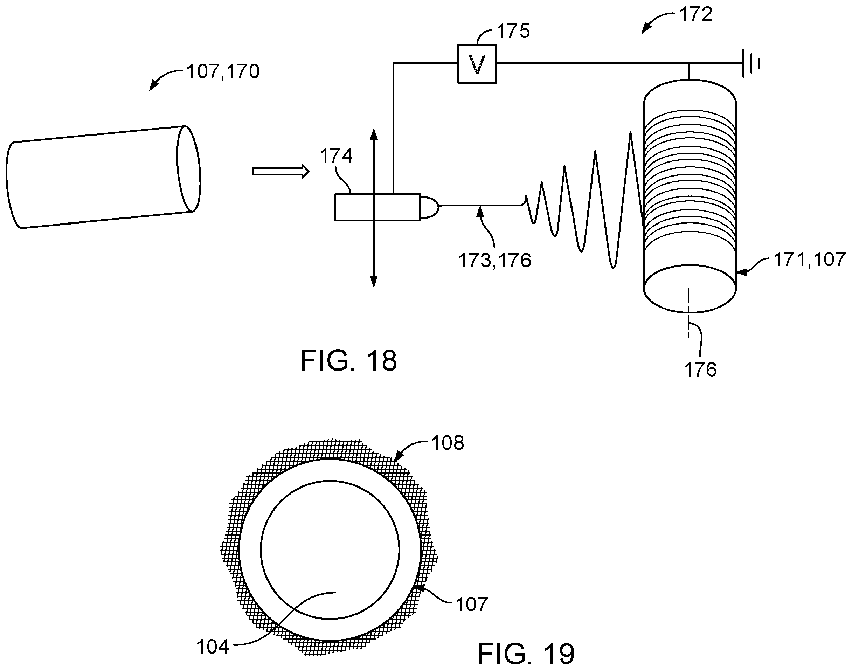

In some embodiments, any of the implantable devices 61, 71, 100, 200, 300, 400, 500, 600 may be manufactured using sequential processes of extruding the interior wall of the container as a tube and then electrospinning the exterior wall of the container as a coating on the tube. For example, referring to FIG. 18 and to the example embodiment of the implantable device 100, the interior wall 107 (e.g., the immune-isolation layer) of the container 102 is produced via an extrusion process 170. In some embodiments, the interior wall 107 is extruded from ePTFE. A porosity of the interior wall 107 (e.g., a void space in the interior wall 107 that depends on a number and size of pores) may be controlled during the extrusion process 170 by manipulating the spacing between nodes and fibrils of the ePTFE. Geometries of fibers of the ePTFE may facilitate subsequent adhesion of the exterior wall 108 of the container 102. However, in some embodiments, a corona treatment (e.g., a type of plasma treatment) may be optionally applied to the interior wall 107 to render the interior wall 107 more suitable for adhesion with the exterior wall 108.

The interior wall 107 is subsequently loaded onto a mandrel 171 of an electrospinning system 172 for application of a fibrous, polymeric material 173 that will be applied to form the exterior wall 108 of the container 102. The mandrel 171 provides a rigid support structure that substantially maintains a shape and a structural integrity of the interior wall 107 while the fibrous material 173 is applied to the interior wall 107.

The electrospinning system 172 also includes a metallic needle 174 for applying the material 173 and a high voltage power supply 175 for powering the metallic needle 174. The mandrel 171, equipped with the interior wall 107 of the container 102, is spun about its central axis 176, and the metallic needle 174 is simultaneously translated parallelly to the central axis 176. While translating, the metallic needle 174 produces and applies a charged jet 177 of the fibrous material 173 to the interior wall 107. The charged jet 177 of the fibrous material 173 coats the interior wall 107 to form the exterior wall 108 (e.g., the vascularization layer) of the container 102, as shown in FIG. 19. In some embodiments, the exterior wall 108 is formed from PVDF. In some embodiments, a porosity of the exterior wall 108 may be controlled by a rotational speed of the mandrel 171, a total time period during which the mandrel 171 is rotated, and environmental conditions, such as ambient temperature and humidity.

Once the exterior wall 108 is formed onto the interior wall 107 to produce the container 102, the spinning is ceased, and the container 102 is removed from the mandrel 171. The first end 105 of the container 102 is sealed, the cells 103 are deposited (e.g., pipetted) into the interior region 104 of the container 102, and the second end 106 of the container 102 is sealed to form the implantable device 100. In some embodiments, the container ends may be sealed using ultra sonic welding or adhesives.

As discussed above with respect to FIG. 3, the exterior wall 108 may be coated with one or more growth factors 117 or other agents that indirectly promote vascularization. Example growth factors 117 include vascular endothelial growth factor (VEGF), placenta derived growth factor (PDGF), transforming growth factor beta (TGFβ), and fibroblast growth factor (FGF). The growth factors 117 or other agents can be coated to the exterior surface 116 of the container 102 by either via non-covalent coating or chemical linkage. The chemical linkage can be formed via random amide coupling using residual amino or carboxylic acid groups in these vascularization-promoting proteins with complementary groups on the surface of the implantable device. Otherwise, these proteins can be linked to the surface via site-specific covalent linkage. The site-specific linkage via thiol-maleimide or thiol-vinyl sulfone addition reaction can be achieved using thiol groups of cysteine amino acids in the proteins and maleimide or vinyl sulfone groups introduced to the exterior surface 116. In addition, by introducing thiol groups to the exterior surface 116, the vascularization-promoting proteins can be linked through disulfide bond using the protein's cysteine residue. Other methods of site-specific conjugation involves oxime bond between carbohydrate residues in the proteins and aminooxy groups on the implantable device surface. Furthermore, by incorporating unnatural amino acids containing specific functional groups as azide groups to the growth factor proteins, either by protein engineering or chemical modifications, these proteins can also be linked to the exterior surface 116 via click chemistry after introducing alkyne groups to the exterior surface 116.

While an example embodiment of the implantable device 100 has been described with respect to FIGS. 18 and 19 as including the interior wall 107 formed of ePTFE and the exterior wall 108 formed of PVDF, in some embodiments, either or both of the interior wall 107 and the exterior wall 108 may be formed of one or more different candidate materials that are mentioned further above.

Additionally, the process described above with respect to FIGS. 18 and 19 for manufacturing the implantable device 100 can be expanded or modified for producing any of the other above-discussed implantable devices. For example, the interior wall 207 of the container 202 and the internal tubes 220, 420 of the implantable devices 200, 400 may be produced using an extrusion process, and the exterior wall 208 of the container 202 may be produced using the above-discussed electrospinning system 172. In some embodiments, the internal tubes 220, 420, 520 of the implantable devices 200, 400, 500 and the outer sleeve 660 of the implantable device 600 are assembled with the container 202 after the container 202 is removed from the mandrel 171 (e.g., after the electro-spinning process has been completed. In some embodiments, the inner tube may be extruded to have features that allow for centering inside of the container 202.

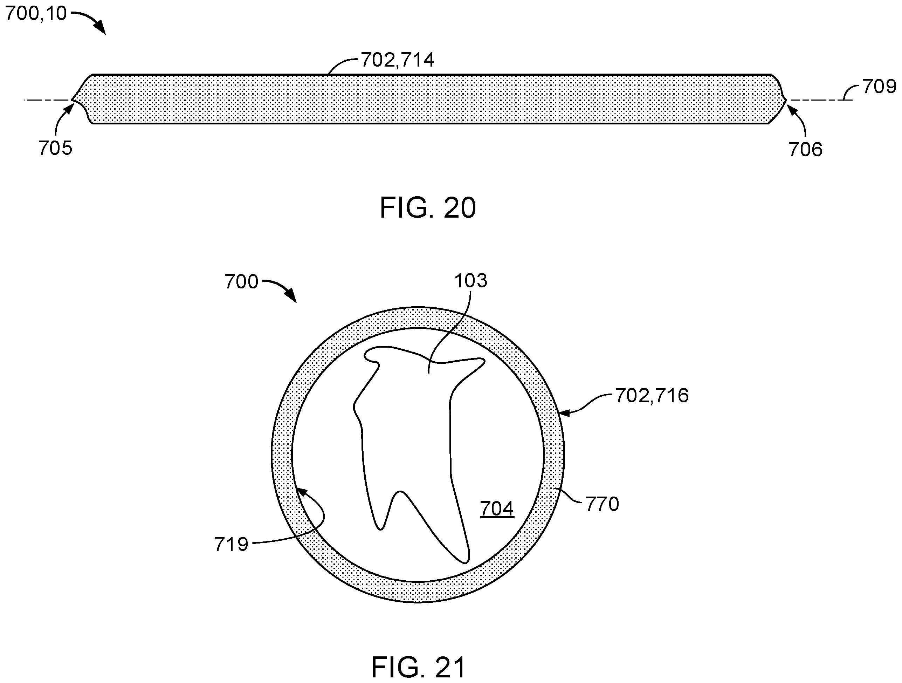

In some embodiments, an implantable device that is substantially similar in function to the implantable device 100 may have a single-layer wall construction with pores that gradually increase in size between interior and exterior surfaces, instead of a double-layer wall construction (e.g., provided by the interior and exterior walls 107, 108) with pores characterized by two discrete size ranges. For example, FIGS. 20 and 21 illustrate an implantable device 700 including a container 702 that has a single-layer wall construction with variable (e.g., gradually changing) pore sizes. Like the implantable device 100, the implantable device 700 is designed to be implanted in a patient's body subcutaneously for providing BCRT therapy to treat diabetes.

The implantable device 700 has a relatively large aspect ratio and may be implanted at the locations within the body that are mentioned above with respect to the implantable device 100. In some embodiments, the implantable device 700 has a surface area to volume ratio of about 200 to about 5,000. The implantable device 700 can typically perform its therapeutic function within the body for a period of about 24 months to about 36 months, and in some cases, even up to a period of about 60 months. The implantable device 700 may be removed and optionally replaced upon losing its capability to perform its therapeutic function.

In addition to the container 702, the implantable device 700 includes multiple cells 103 (e.g., human beta cells) that are located within an interior region 704 (e.g., a cell reservoir) of the container 702. The container 702 has a generally tubular shape that provides the implantable device 700 with the linear configuration 10. The container 702 is closed (e.g., sealed) at opposite ends 705, 706 to maintain the cells 103 within the interior region 704. The container 702 defines an interior surface 719 and an exterior surface 716. The container 702 can be further described with respect to an interior wall portion 707 that extends radially outward from the interior surface 719, an exterior wall portion 708 that extends radially inward from the exterior surface 716, and an intermediate wall portion 771 that extends radially between the interior and exterior wall portions 707, 708. In some embodiments, the exterior wall portion 708 may account for approximately the outer radial half of container 702. In some embodiments, the interior wall portion 707 may account for approximately the inner radial quarter of container 702.

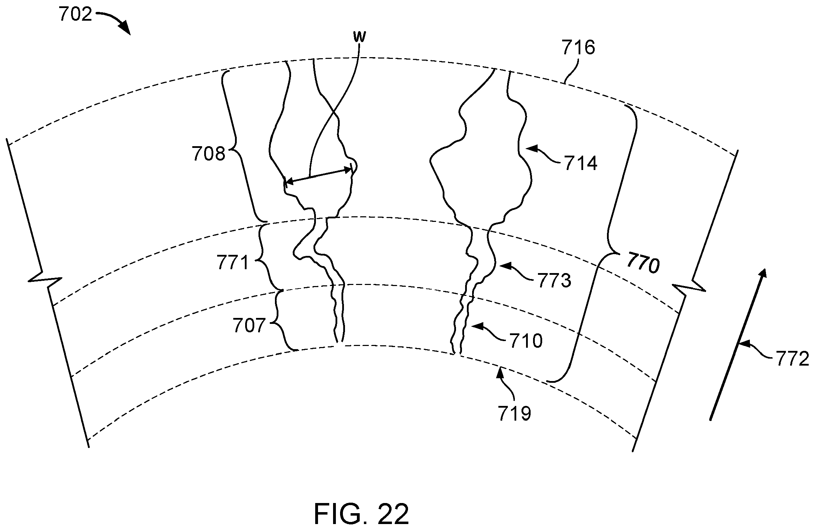

Referring to FIG. 22, features of the wall portions 707, 708, 771 determine significant functional capabilities of the implantable device 700. For example, the container 702 defines pores 770 (e.g., void regions lacking material) that gradually increase in size along a radially outward direction 772 between the interior surface 719 and the exterior surface 716. The pores 770 may have cross-sectional shapes that are generally ovular, elongate, and asymmetric.

In particular, the container 702 defines pores 710 within the interior wall portion 707 that are sized to allow passage of the nutrients 111 needed by the cells 103 to remain viable and to produce insulin 101. The pores 710 are also sized to allow passage of insulin 101 and the waste products 112 produced by the cells 103. Accordingly, the nutrients 111 pass into the interior region 704 of the container 702, while the insulin 101 and the waste products 112 pass out of the interior region 704, as generally illustrated with respect to the implantable device 100 in FIG. 2.

The pores 710 are also sized to prevent passage of the cells 103 out of the interior wall portion 707. Therefore, along with closure of the ends 705, 706 of the container 702, the pores 710 are responsible for containing the cells 103 within the container 102. Importantly, the pores 710 are also sized to prevent passage of the components 113 of the patient's immune system into the interior region 704 of the container 702 to isolate and protect the cells 103 from attack by the components 113. Accordingly, the pores 710 of the interior wall portion 707 provide the implantable device 700 with the functional capability of immunoisolation for protecting the cells 103 from the immune system. Within the interior wall portion 707, the pores 710 gradually increase in size in the direction 772 and typically have widths w in a range of about 10 nm to about 400 nm. Sizes of the pores 710 can be measured or confirmed using the characterization techniques described above with respect to the pores 110. The pores 710 can provide the above-mentioned functionalities across this full range of widths.

The container 702 defines pores 714 within the exterior wall portion 708. Because the pores 714 are larger than the pores 710 within the interior wall portion 707, the pores 714 permit passage of the nutrients 111, the insulin 101, and the waste products 112. Thus, pores 714 along the exterior surface 716 of the container 702 are in fluid communication with pores 710 along the interior surface 719 of the container 702. The pores 714 also allow passage of the components 113 of the immune system. However, these components 113 are ultimately rejected from the interior region 704 of the container 702 by the pores 710 within the interior wall portion 707. Importantly, the pores 714 are sized to promote vascularization and oxygen transfer along the exterior surface 716 of the container 702, as discussed above with respect to the pores 114 of the implantable device 100. Within the exterior wall portion 708, the pores 714 gradually increase in size in the direction 772 and typically have widths w in a range of about 2 µm to about 60 µm. In some embodiments, the pores 714 may have an average width of about 5 µm, and the pore sizes may be measured using the techniques described above with respect to the pores 110. The pores 714 can provide the above-mentioned functionalities across this full range of widths.

Between the interior and exterior wall portions 707, 708, the container 702 defines pores 773 within the intermediate wall portion 771. Within the intermediate wall portion 771, the pores 773 gradually increase in size in the direction 772 and have widths w, and the pore sizes may be measured or confirmed using the techniques described above with respect to the pores 110. Depending on an intermediate size of each pore 773, some of the pores 773 may provide functionalities of the pores 710, whereas other pores 773 may provide functionalities of the pores 714.

Additionally, the exterior surface 716 of the container 702 may also be coated with one or more of the growth factors 117 or other agents that indirectly promote vascularization and that are discussed above with respect to the implantable device 100. As a result of either or both of the sizes of the pores 714 and the one or more growth factors 117 or other agents, the implantable device 700 is typically vascularized to an extent such that blood vessels 115 can continually provide adequate concentrations of the nutrients 111 within a period of about two weeks to about three weeks following implantation. Blood flowing through the blood vessels 115 delivers the nutrients 111 to the implantable device 700 and takes up the insulin 101 and the waste products 112 secreted by the cells 103 within the interior region 704 of the container 702.

The cells 103 are present within the interior region 704 of the container 702 in an amount that is sufficient to produce a therapeutic effect. For example, the interior region 704 of the container 702 typically has a volume capacity of about 0.1 mL to about 1.1 mL.

Moreover, the cells 103 are arranged within the container 702 in a manner such that each of the cells 103 can receive a sufficient amount of oxygen from passive diffusion of the oxygen through the pores 770 alone and without an additional energy source for providing supplemental oxygen to the cells 103, as discussed above with respect to the implantable device 100. Accordingly, a width (e.g., a diameter) of the interior region 704 of the container 702 is limited to accommodating a single cell 103 along a central axis 709 of the container 702, as shown in FIG. 20. For example, the interior region 704 typically has a width in a range of about 100 µm to about 2 mm.

As discussed above with respect to the implantable device 100, the implantable device 700 has a relatively low, narrow profile, a tubular shape, and a size that maximizes a ratio of external surface area to volume for providing the functional capability of passive oxygenation for the cells 103. For example, the container 702 typically has an external surface area to volume ratio that is typically between about 1,000 and about 10,000. The container 702 typically has a wall thickness (e.g., a sum of the thicknesses of the interior, intermediate, and exterior wall portions 707, 771, 708) in a range of about 1 µm to about 2 µm such that the implantable device 700 has a total width in a range about 0.1 mm to about 2 mm. The implantable device 700 typically has a length in a range about 1 m to about 60 m. Accordingly, the implantable device 700 is embodied as a long, thin tube.

Furthermore, the container 702 of the implantable device 700 is a flexible structure. The flexibility of the container 702 facilitates positioning of the implantable device 700 within asymmetric spaces of the body. In some embodiments, the container 102 may be made of one or more HFM materials, such as PCL, PTFE, ePTFE, nylon, polyether-ketone, polyether sulfone, polyester, PVDF, and polysiloxane.

In some embodiments, the implantable device 700 may be adjusted (e.g., bent, biased, or otherwise deformed) from the linear configuration 10 shown in FIG. 20 into a round, curved, or otherwise non-linear configuration, such as the spiral and helical configurations 20, 30 shown in FIGS. 4-6, and may be maintained in such a configuration by meshed or molded support structure to which the implantable device 700 may be attached. In the spiral configuration 20 or helical configuration 30, the implantable device 700 may be arranged, sized, or otherwise dimensioned as described above with respect to like configurations of the implantable device 100.

In some embodiments, multiple implantable devices 700 may be utilized together or coupled to one another to form an implantable system with a parallel arrangement 41 or a matrix arrangement 51, such as illustrated for the implantable systems 40, 50 including the implantable device 100. For the parallel arrangement 41 or the matrix arrangement 51, the implantable device 700 may be arranged, sized, or otherwise dimensioned as described above with respect to like configurations of the implantable device 100.

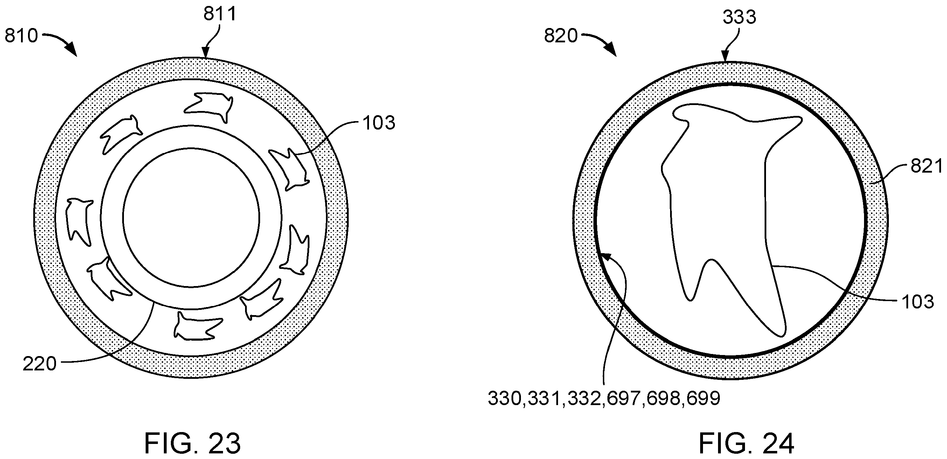

In some embodiments, an implantable device that is designed for passive oxygenation or, in comparative examples, an implantable device that is equipped with a substance to provide active oxygenation and that is otherwise substantially similar in construction and function to any of the implantable devices 200, 300, 400, 500, 600 may include an outer container that is alternatively formed with the single-layer wall construction described above with respect to the container 702 instead of the double-layer wall construction of the containers 102, 202. For example, FIG. 23 illustrates an implantable device 810 that is substantially similar in construction and function to the implantable device 200, except that the implantable device 810 includes a container 811 with a single-layer wall construction instead of the container 202. Accordingly, the implantable device 810 also includes the internal tube 220 and cells 103. The container 811 has the single-layer wall construction with gradually varying pore sizes as described above with respect to the container 702, but is otherwise similar in structure and function to the container 202.

FIG. 24 illustrates an implantable device 820 that is substantially similar in construction and function to the implantable device 300, except that the implantable device 820 includes a container 821 with a single-layer wall construction instead of the container 102. Accordingly, the implantable device 820 also includes cells 103, the hydrophobic polymer 333, and the coating 332 of the particles 331 encapsulating the substance 330. The container 821 has the single-layer wall construction with gradually varying pore sizes as described above with respect to the container 702, but is otherwise similar in structure and function to the container 102.

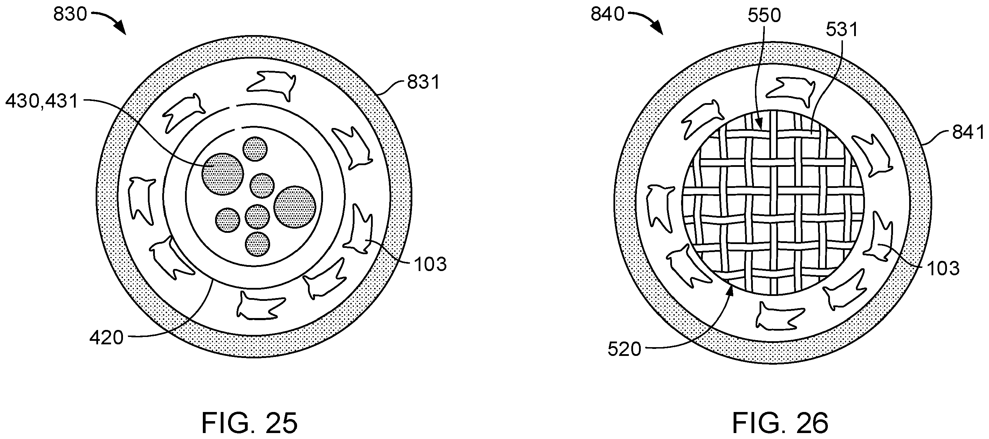

FIG. 25 illustrates an implantable device 830 that is substantially similar in construction and function to the implantable device 400, except that the implantable device 830 includes a container 831 with a single-layer wall construction instead of the container 202. Accordingly, the implantable device 830 also includes cells 103, the internal tube 420, and the particles 431 encapsulating the substance 430. The container 831 has the single-layer wall construction with gradually varying pore sizes as described above with respect to the container 702, but is otherwise similar in structure and function to the container 202.

FIG. 26 illustrates an implantable device 840 that is substantially similar in construction and function to the implantable device 500, except that the implantable device 840 includes a container 841 with a single-layer wall construction instead of the container 202. Accordingly, the implantable device 840 also includes cells 103, the internal tube 520, and the substance 530 loaded within the sintered matrix 550. The container 841 has the single-layer wall construction with gradually varying pore sizes as described above with respect to the container 702, but is otherwise similar in structure and function to the container 202.

In some embodiments, any of the implantable devices 700, 810, 820, 830, 840 may be equipped with the outer sleeve 660 formed of the sintered matrix 650, provided with the cuff 66 as part of a therapeutic system, or provided with the transdermal patch 77 as part of a therapeutic system.

In some embodiments, any of the implantable devices 820, 830, 840, 860 may further include additional particles 697 that encapsulate a catalyst 698 and that are applied to the inner surface 719 of the interior wall portion 707 as part of a coating 699, as discussed above with respect to the implantable devices 300, 400, 500, 600, and as illustrated in FIG. 24. In some embodiments, the substance 330, 430, 530, 630 may be hydrogen peroxide, and the catalyst 698 may be catalase (e.g., or any of the other above-discussed catalysts), both present at the concentrations discussed above.

In some embodiments, the implantable devices 700, 810, 820, 830, 840 may be manufactured using a well-controlled phase separation technique, such as non-solvent induced phase separation (NIPS). According to a NIPS protocol, a flat non-woven substrate (e.g., a sheet) may be rolled into a tube, ends of the tube may be closed (e.g., sealed), and the tube may be exposed to (e.g., immersed in) a solvent. In some embodiments, the tube may be made of one or more HFM materials, such as polycaprolactone, PTFE, ePTFE, nylon, polyether-ketone, polyether sulfone, polyester, polyvinylidene difluoride, and polysiloxane. Referring to the example embodiment of the implantable device 700, the solvent gradually degrades the material of the tube along the exterior surface 716 to form the pores 770 of various sizes, resulting in the container 702.

Sizes of the pores 770 increase proportionally to an exposure time, such that the pores 714 within the exterior wall portion 708 have larger sizes than those of the pores 710 within the interior wall portion 707. In some embodiments, a porosity gradient of the container 702 can be controlled via selection of the solvent and a coagulation time. Example solvents that may be used to carry out the NIPS protocol include noprotic solvents of different polarities, such as N-methyl pyrrolidone, N,N-dimethyl acetamide, tetrahydrofuran, toluene, DMF, tetramethyl urea, methyl ethyl ketone, supercritical CO2,etc. In some embodiments, sizes of the pores 770 may be controlled by injecting additives into the solvent. Example additives include polyvinylpyrolidone, polyethylene glycol, polymethyloxazoline, poly(N,N-dimethyl) (meth)acrylamide, etc.

The container 102 can be subsequently coated with the growth factor 117 or any of the other above-discussed agents that indirectly promote vascularization. Example growth factors 117 include vascular endothelial growth factor (VEGF), placenta derived growth factor (PDGF), transforming growth factor beta (TGFβ), and fibroblast growth factor (FGF). As discussed above, the growth factors 117 or other agents can be coated to the exterior wall portion 708 of the container 102 by either via non-covalent coating or chemical linkage. Once the container 702 is formed, one end of the container is opened, the cells 103 are deposited into the interior region 704 of the container 702, and the end of the container 702 is re-sealed to form the implantable device 700. In some embodiments, the container ends may be sealed (e.g., or resealed) using ultra sonic welding or adhesives.

While the implantable devices 700, 810, 820, 830, 840 have been described as having a gradually varying pore size that is achieved by carrying out a NIPS protocol, in some embodiments, an implantable device that is otherwise similar in construction and function to the implantable device 700 may alternatively be manufactured according to a different phase separation technique or another type of manufacturing process to achieve a gradually varying pore size. For example, such an implantable device may be produced according to a thermally induced phase separation (TIPS) technique, a vapor-induced phase separation (VIPS) technique, or a NIPS protocol that is followed by separation micromolding (PSµM). In some embodiments, such an implantable device may include a multi-layer wall construction with gradually varying pore sizes. For example, the implantable device may include a substrate formed via TIPS, a next layer formed via NIPS, and a final layer formed by interfacial polymerization (IP). In other embodiments, such an implantable device may be formed according to a 3D printing protocol that includes stages of photo-polymerization, lamination, powder jetting, and extrusion printing.

While the interior regions and the annular lumens of the above-discussed implantable devices 100, 200, 700, 810 have been described and illustrated as having a width that is limited to accommodating a single cell 103, in some comparative examples, an implantable device that is otherwise substantially similar in construction and function to any of the implantable devices 100, 200, 700, 810 may have an interior region or an annular lumen that is substantially wider than a width of a single cell 103, but that includes cells 103 at a low enough concentration to ensure spreading out of the cells 103 and sufficient oxygenation of the cells 103 solely via passive diffusion during the vascularization period. An example of such an implantable device 850 that includes cells 103 within an interior region 855 of a container 851 is illustrated in FIG. 26.

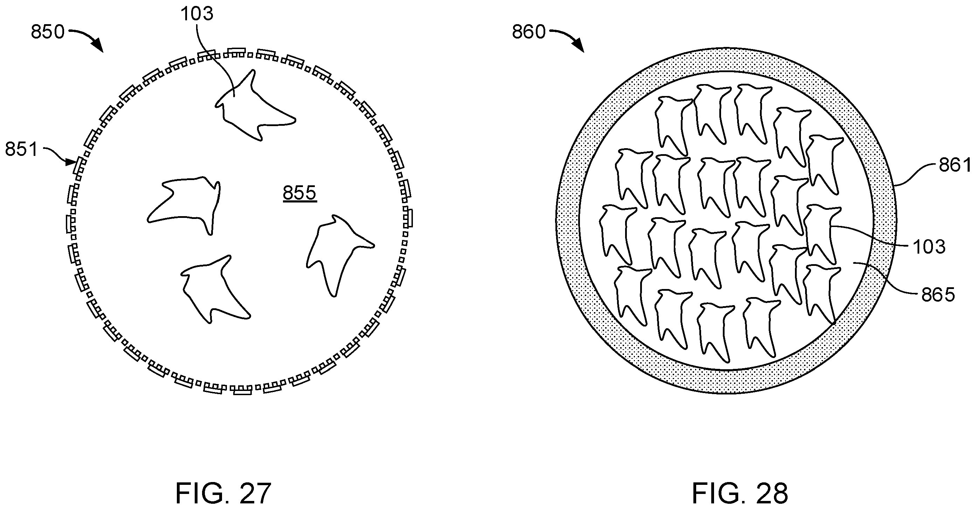

While the interior regions and the annular lumens of the above-discussed implantable devices 300, 400, 500, 600, 820, 830, 840 have been described and illustrated as having a width that is limited to accommodating a single cell 103, in some comparative examples, an implantable device that is otherwise substantially similar in construction and function to any of the implantable devices 300, 400, 500, 600, 820, 830, 840 may have an interior region or an annular lumen that is substantially wider than a width of a single cell 103 to accommodate a higher cell density and therefore include an active oxygenation configuration that is suitable for providing supplemental oxygen over a long-term period of about two years to about three years. For example, such an implantable device may include an interior region or an annular lumen that is wide enough to accommodate multiple cells 103 across an axial section of the interior region or annular lumen. An example of such an implantable device 860 that includes cells 103 within an interior region 865 of a container 861 is illustrated in FIG. 27.

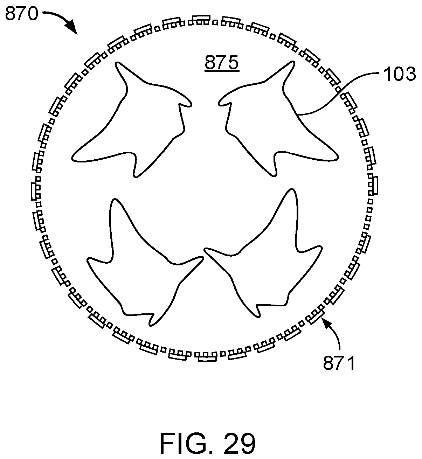

In some embodiments, an implantable device with a double-layer wall construction substantially similar to that of the implantable device 100 or with a single-layer wall construction substantially similar to that of the implantable device 700 may be provided with a single-layer circumferential arrangement of cells. Such an implantable device therefore includes multiple cells per axial unit of length, but without an inner tube that imposes the arrangement. The implantable device therefore avoids any structural, manufacturing, and physiological complexities associated with including such an inner tube. An example of such an implantable device 870 that includes cells 103 within an interior region 875 of a container 871 is illustrated in FIG. 29.

While the above-discussed implantable devices have been described as treatments for type 1 diabetes, type 2 diabetes, and hemophilia, in some embodiments, any of the above-discussed implantable devices may alternatively or additionally provide treatment for other diseases, such as growth hormone deficiencies (e.g., Fabry disease, Pompe disease, mucopolysaccharidosis type I, Niemann-Pick disease type A, Niemann-Pick disease type B, and phenylketonuria).

While the above-discussed implantable devices have been described as BCRT implants including human beta cells that produce insulin to treat diabetes, in some embodiments, an implantable device that is otherwise substantially similar in construction and function to any of the above-discussed implantable devices may alternatively include a different type of cell that produces a different type of therapeutic agent (e.g., any of the therapeutic agents described herein) to treat a different disease (e.g., any of the diseases described herein).

In general, the therapeutic agent is one of various types of proteins, such as an antibody or an antibody fragment, an enzyme, an engineered protein, or a protein fragment. In embodiments for which the therapeutic agent is an engineered protein, the engineered protein may be a hormone, such as insulin or an insulin analog. In some embodiments, the engineered protein may be a cytokine. In some embodiments, the engineered protein may be coagulation factor VIIa, coagulation factor VIII, coagulation factor IX, follicle stimulating hormone (FSH), anti-thrombin III, albumin, erythropoietin, granulocyte colony stimulating factor (GCSF), granulocyte macrophage colony stimulating factor (GMCSF), interleukin 2, interleukin 11 (IL-11), interleukin 12 (IL-12), (VEGF), insulin, Glucagon-like peptide 1 (GLP-1), glucose-dependent insulinotropic polypeptide (GIP), glucocerebrosidase, agalisidase beta, alglucosidase alpha, laronidase, idursulfase, acid sphingomyelinase, phenylalanine hydroxylase , glucose-6-phosphatase, C-type natriuretic peptide, interferon beta, relaxin, human growth hormone, or parathyrin.

A variety of different methods known in the art can be used to generate a cell that can produce a therapeutic agent. Non-limiting examples of such methods include: lipofection, transfection (e.g., calcium phosphate transfection, transfection using highly branched organic compounds, transfection using cationic polymers, dendrimer-based transfection, optical transfection, particle-based transfection (e.g., nanoparticle transfection), or transfection using liposomes (e.g., cationic liposomes)), microinjection, electroporation, cell squeezing, sonoporation, hydrodynamic delivery, impalefection, gene gun, magnetofection, viral transfection, and nucleofection. Various molecular biology techniques are known in the art that can be used to introduce a mutation(s) and/or deletion(s) into an endogenous gene in order to express and/or over-express a therapeutic agent (e.g., any of the therapeutic agents described herein). Non-limiting exampes of such techniques include: site-directed mutagenesis, CRISPR (e.g., CRISPR/Cas9-induced knock-in mutations and CRISPR/Cas9-induced knockout mutations), and TALENS. Any of the therapeutic agents described herein can also be expressed by a vector (e.g., an expression vector, a plasmid (e.g., naked or contained in liposomes), a transposon, an artificial chromosome, or a viral vector (e.g., an adeno-associated virus (AAV) vector, an adenovirus vector, a lentivirus vector or a retrovirus vector)). Any of the vectors described herein can further include a control sequence, e.g., a control sequence selected from the group consisting of a transcription initiation sequence, a transcription termination sequence, a promoter sequence (e.g., a constitutive promoter, an inducible promoter, and/or a tissue specific promoter), an enhancer sequence, an RNA splicing sequence, a polyadenylation (polyA) sequence, an internal ribosome entry site (IRES) sequence, and a Kozak consensus sequence. Additional features of a vector are known to those skilled in the art, and can be included in any of the vectors described herein. Skilled practitioners will be capable of selecting suitable vectors and cells (e.g., mammalian cells (e.g., beta cells (e.g., human beta cells))) for producing a cell that produces any of the therapeutic agents described herein.

While the above-discussed implantable devices have been described and illustrated with respect to certain dimensions, sizes, shapes, arrangements, configurations, materials, components, and methods, in some embodiments, an implantable device that is otherwise substantially similar in construction and function to any of the above-discussed implantable devices may include one or more different dimensions, sizes, shapes, arrangements, configurations, materials, and components, or may be manufactured according to different methods. Accordingly, other embodiments are also possible as long as they are within the scope of the following claims.

## Claims

1. An implantable device (100, 700) for providing a therapeutic agent (101), the implantable device comprising:
a container (102, 702,) configured to contain a plurality of cells (103) capable of producing the therapeutic agent within an interior region (104, 704) of the container, the container defining:
first pores (110, 710) defined by an interior wall portion (107, 707) of the container, the first pores having a first average size that (i) allows passage of the therapeutic agent and oxygen (111) through the first pores and (ii) prevents passage of immune cells (113) through the first pores, and
second pores (114, 714) defined by an exterior wall portion (108, 708) of the container, the second pores having a second average size that is larger than the first average size, and the second pores being sized to promote vascularization (115) and oxygen transfer along the exterior wall portion,
wherein the container has a generally tubular shape, wherein the interior region of the container has a width that is limited to accommodating a single cell (103) of the plurality of cells along a central axis (109, 709) of the container, such that at least a portion of a surface area of each cell of the plurality of cells (103) has direct exposure to the first pores of the interior wall portion, such that the plurality of cells is arrangeable within the container to receive a sufficient amount of oxygen for survival of the plurality of cells from passive diffusion of the oxygen through the first and second pores without an additional energy source for providing supplemental oxygen to the plurality of cells, and
wherein the container comprises a tube, and wherein the tube has a spiral configuration (20) or a helical configuration (30).

2. The implantable device (100, 700) of claim 1, wherein the implantable device has an external surface area to volume ratio of about 200 to about 5,000.

3. The implantable device (100, 700) of any of claims 1 and 2, wherein the second average size of the second pores is configured to allow passage of the therapeutic agent (101) through the exterior wall portion (108, 708) to vasculature adjacent the implantable device.

4. The implantable device (100, 700) of any of the preceding claims, wherein the first pores (110, 710) have first widths in a range of about 10 nm to about 400 nm, and wherein the second pores (114, 714) have second widths in a range of about 2 µm to about 60 µm.

5. The implantable device (100, 700) of any of the preceding claims,
wherein the container (102, 702) has a wall thickness in a range of 1 µm to 100 µm; and
wherein the interior region (104, 704) has a width in a range of about 100 µm to about 2 mm.

6. The implantable device (100, 700) of any of the preceding claims, wherein the interior region (104, 704) has a volume capacity of about 0.1 mL to about 1.5 mL or a volume capacity of about 0.1 mL to about 1.1 mL.

7. The implantable device (100, 700) of any of the preceding claims, wherein the exterior wall portion (108, 708) is coated with a growth factor (117) that promotes vascularization (115), and:
wherein the growth factor is covalently linked to the exterior wall portion, and/or
wherein the growth factor is electrostatically bound to the exterior wall portion, and/or
wherein the growth factor is site-specifically bound to the exterior wall portion, and/or
wherein the growth factor is selected from the group consisting of: vascular endothelial growth factor (VEGF), placenta derived growth factor (PDGF), transforming growth factor beta (TGFβ), and fibroblast growth factor (FGF), and/or
wherein the growth factor is vascular endothelial growth factor (VEGF), placenta derived growth factor (PDGF), transforming growth factor beta (TGFβ), fibroblast growth factor (FGF), or a combination thereof, and/or
wherein the growth factor is vascular growth factor (VEGF).

8. The implantable device (100, 700) of any of the preceding claims, further comprising the plurality of cells (103) contained within the interior region (104, 704) of the container (102, 702).

9. The implantable device (100, 700) of claim 8, wherein the plurality of cells (103) are beta cells, and wherein the therapeutic agent (101) comprises insulin.

10. The implantable device (100, 700) of any of the preceding claims, further comprising one or more additional containers (40, 50) associated with the container (102, 702), wherein each of the one or more additional containers contains an additional plurality of cells (103).

11. The implantable device (700) of any of the preceding claims, wherein the container comprises a tubular wall (702) that comprises the interior (107, 707) and exterior wall portions (108, 708), wherein the tubular wall further defines third pores (773) arranged radially between the first and second pores, wherein the third pores have a third average size that is greater than the first average size and less than the second average size, wherein the tubular wall defines pores (770) formed by the first, third, and second pores such that the pores (770) gradually increase in size along a radially outward direction (772) from an interior surface (719) of the tubular wall to an exterior surface (716) of the tubular wall, and wherein the container comprises one or more of polycaprolactone, PTFE, ePTFE, nylon, polyether-ketone, polyether sulfone, polyester, polyvinylidene difluoride, and polysiloxane.

12. The implantable device of any of claims 1-10, wherein the interior wall portion comprises a tubular member (107) and the exterior wall portion comprises a coating (108, 173) that surrounds the tubular member, wherein the tubular member has a first material formulation and the coating has a second material formulation that is different from the first material formulation, wherein the first material formulation comprises one or more of expanded polytetrafluoroethylene (ePTFE), mixed cellulose ester, polyethersulfone (PES), modified PES, alginate, polyethylene glycol (PEG), polyvinylpyrrolidone, poly(methylene-co-guanidine), polyvinyl alcohol, copolymer of vinyl pyrrolidone, hydroxypropyl methacylamide, hydroxypropyl methacrylate, hydroxyethyl methacrylate, poly(oxazolines), hyaluronic acid, polyoxazoline, polyhydroxypropylmethacrlamide, zwiterionic polymers, and polymers containing carboxybetaine, sulfobetaine, and phosphoryl choline groups, and wherein the second material formulation comprises one or more of polyvinylidene difluoride (PVDF), polycaprolactone (PCL), nylon (e.g., nylon-6), polytetrafluoroethylene (PTFE), ePTFE, polyether-ketone, polyether sulfone, polyester, polysiloxane, polyether ketone, poly(vinylidine fluoride-co-hexafluropropylene), cellulose acetate, and polypropylene.

13. A therapeutic system (60, 70) comprising:
the implantable device (100, 600, 700) of any one of claims 1-12, the implantable device configured to be implanted subcutaneously along a skin region (63, 73); and
an accessory device (66, 77, 660) configured to cooperate with the implantable device for promoting delivery of oxygen to the plurality of cells.

14. The therapeutic system (60, 70) of claim 13, wherein the accessory device (66, 77, 660) comprises:
(i) a transdermal patch (77) that is configured to be secured to an exterior skin surface of the skin region (73), the transdermal patch comprising a plurality of needles (79) that carry a substance capable (74) of reacting to generate oxygen (75) for the plurality of cells (103) within the interior region (104, 704) of the container, or
(ii) a cuff (66) that is configured to be secured to and apply pressure to the exterior skin surface of the skin region (63) to direct a flow of blood (64) towards the implantable device (100, 600, 700), or
(iii) a sintered mesh (650) that surrounds the implantable device and that carries a substance capable (630) of reacting to generate oxygen.

## Patentansprüche

1. Implantierbare Vorrichtung (100, 700) zur Bereitstellung eines therapeutischen Mittels (101), wobei die implantierbare Vorrichtung umfasst:
einen Behälter (102, 702), gestaltet zum Enthalten einer Vielzahl von Zellen (103), die fähig sind, das therapeutische Mittel in einem Innenbereich (104, 704) des Behälters zu erzeugen, wobei der Behälter definiert:
erste Poren (110, 710), definiert von einem inneren Wandabschnitt (107, 707) des Behälters, wobei die ersten Poren eine erste mittlere Größe aufweisen, die (i) Durchgang des therapeutischen Mittels und von Sauerstoff (111) durch die ersten Poren erlaubt und (ii) Durchgang von Immunzellen (113) durch die ersten Poren verhindert, und
zweite Poren (114, 714), definiert von einem äußeren Wandabschnitt (108, 708) des Behälters, wobei die zweiten Poren eine zweite mittlere Größe aufweisen, die größer als die erste mittlere Größe ist, und wobei die zweiten Poren bemessen sind, Vaskularisation (115) und Sauerstoffüberführung entlang des äußeren Wandabschnitts zu fördern;
wobei der Behälter eine allgemein rohrförmige Form aufweist, wobei der Innenbereich des Behälters eine Weite aufweist, die auf die Aufnahme einer einzelnen Zelle (103) der Vielzahl von Zellen entlang einer Mittelachse (109, 709) des Behälters beschränkt ist, so dass wenigstens ein Teil eines Oberflächenbereichs jeder Zelle der Vielzahl von Zellen (103) gegenüber den ersten Poren des inneren Wandabschnitts direkt exponiert ist, so dass die Vielzahl von Zellen innerhalb des Behälters angeordnet werden können, um eine ausreichende Menge an Sauerstoff für das Überleben der Vielzahl von Zellen durch passive Diffusion des Sauerstoffs durch die ersten und zweiten Poren ohne eine zusätzliche Energiequelle zum Bereitstellen von zusätzlichem Sauerstoff für die Vielzahl von Zellen aufzunehmen, und
wobei der Behälter ein Rohr umfasst und wobei das Rohr eine spiralförmige Konfiguration (20) oder eine helikale Konfiguration (30) aufweist.

2. Implantierbare Vorrichtung (100, 700) nach Anspruch 1, wobei die implantierbare Vorrichtung ein Verhältnis von Außenfläche zu Volumen von etwa 200 bis etwa 5.000 aufweist.

3. Implantierbare Vorrichtung (100, 700) nach einem der Ansprüche 1 und 2, wobei die zweite mittlere Größe der zweiten Poren dafür gestaltet ist, Durchgang des therapeutischen Mittels (101) durch den äußeren Wandabschnitt (108, 708) zu Gefäßen benachbart zu der implantierbaren Vorrichtung zu erlauben.

4. Implantierbare Vorrichtung (100, 700) nach einem der vorstehenden Ansprüche, wobei die ersten Poren (110, 710) erste Weiten in einem Bereich von etwa 10 nm bis etwa 400 nm aufweisen und wobei die zweiten Poren (114, 714) zweite Weiten in einem Bereich von etwa 2 µm bis etwa 60 µm aufweisen.

5. Implantierbare Vorrichtung (100, 700) nach einem der vorstehenden Ansprüche,
wobei der Behälter (102, 702) eine Wandstärke in einem Bereich von 1 µm bis 100 µm aufweist; und
wobei der Innenbereich (104, 704) eine Weite in einem Bereich von etwa 100 µm bis etwa 2 mm aufweist.

6. Implantierbare Vorrichtung (100, 700) nach einem der vorstehenden Ansprüche, wobei der Innenbereich (104, 704) eine Volumenkapazität von etwa 0,1 ml bis etwa 1,5 ml oder eine Volumenkapazität von etwa 0,1 ml bis etwa 1,1 ml aufweist.

7. Implantierbare Vorrichtung (100, 700) nach einem der vorstehenden Ansprüche, wobei der äußere Wandabschnitt (108, 708) mit einem Wachstumsfaktor (117) beschichtet ist, der Vaskularisation (115) fördert, und:
wobei der Wachstumsfaktor kovalent an den äußeren Wandabschnitt gebunden ist, und/oder
wobei der Wachstumsfaktor elektrostatisch an den äußeren Wandabschnitt gebunden ist, und/oder
wobei der Wachstumsfaktor ortsspezifisch an den äußeren Wandabschnitt gebunden ist, und/oder
wobei der Wachstumsfaktor ausgewählt ist aus der Gruppe bestehend aus:
vaskulärem endothelialem Wachstumsfaktor (VEGF), Plazenta-abgeleitetem Wachstumsfaktor (PDGF), transformierendem Wachstumsfaktor beta (TGFβ) und Fibroblasten-Wachstumsfaktor (FGF) und/oder
wobei der Wachstumsfaktor vaskulärer endothelialer Wachstumsfaktor (VEGF), Plazenta-abgeleiteter Wachstumsfaktor (PDGF), transformierender Wachstumsfaktor beta (TGFβ), Fibroblasten-Wachstumsfaktor (FGF) oder eine Kombination davon ist, und/oder
wobei der Wachstumsfaktor vaskulärer Wachstumsfaktor (VEGF) ist.

8. Implantierbare Vorrichtung (100, 700) nach einem der vorstehenden Ansprüche, ferner umfassend die Vielzahl von Zellen (103), die in dem Innenbereich (104, 704) des Behälters (102, 702) enthalten sind.

9. Implantierbare Vorrichtung (100, 700) nach Anspruch 8, wobei die Vielzahl von Zellen (103) Betazellen sind und wobei das therapeutische Mittel (101) Insulin umfasst.

10. Implantierbare Vorrichtung (100, 700) nach einem der vorstehenden Ansprüche, ferner umfassend einen oder mehrere zusätzliche Behälter (40, 50), die mit dem Behälter (102, 702) verbunden sind, wobei jeder des einen oder der mehreren zusätzlichen Behälter eine zusätzliche Vielzahl von Zellen (103) enthält.

11. Implantierbare Vorrichtung (700) nach einem der vorstehenden Ansprüche, wobei der Behälter eine rohrförmige Wand (702) umfasst, die den inneren (107, 707) und den äußeren Wandabschnitt (108, 708) umfasst, wobei die rohrförmige Wand ferner dritte Poren (773) definiert, die radial zwischen den ersten und zweiten Poren angeordnet sind, wobei die dritten Poren eine dritte mittlere Größe aufweisen, die größer als die erste mittlere Größe und kleiner als die zweite mittlere Größe ist, wobei die rohrförmige Wand Poren (770) definiert, die von den ersten, dritten und zweiten Poren gebildet werden, so dass die Poren (770) entlang einer radial nach außen gerichteten Richtung (772) von einer Innenfläche (719) der rohrförmigen Wand zu einer Außenfläche (716) der rohrförmigen Wand allmählich in Größe zunehmen, und wobei der Behälter eines oder mehrere von Polycaprolacton, PTFE, ePTFE, Nylon, Polyetherketon, Polyethersulfon, Polyester, Polyvinylidendifluorid und Polysiloxan umfasst.

12. Implantierbare Vorrichtung nach einem der Ansprüche 1-10, wobei der innere Wandabschnitt ein rohrförmiges Element (107) umfasst und der äußere Wandabschnitt eine Beschichtung (108, 173) umfasst, die das rohrförmige Element umgibt, wobei das rohrförmige Element eine erste Materialformulierung aufweist und die Beschichtung eine zweite Materialformulierung aufweist, die von der ersten Materialformulierung verschieden ist, wobei die erste Materialformulierung eines oder mehrere von expandiertem Polytetrafluorethylen (ePTFE), gemischtem Celluloseester, Polyethersulfon (PES), modifiziertem PES, Alginat, Polyethylenglykol (PEG), Polyvinylpyrrolidon, Poly(methylen-co-guanidin), Polyvinylalkohol, Copolymer von Vinylpyrrolidon, Hydroxypropylmethacrylamid, Hydroxypropylmethacrylat, Hydroxyethylmethacrylat, Poly(oxazolinen), Hyaluronsäure, Polyoxazolin, Polyhydroxypropylmethacrylamid, zwitterionischen Polymeren und Polymeren, die Carboxybetain-, Sulfobetain- und Phosphorylcholingruppen umfassen, umfasst, und wobei die zweite Materialformulierung eines oder mehrere von Polyvinylidendifluorid (PVDF), Polycaprolacton (PCL), Nylon (z.B. Nylon-6), Polytetrafluorethylen (PTFE), ePTFE, Polyetherketon, Polyethersulfon, Polyester, Polysiloxan, Polyetherketon, Poly(vinylidinfluorid-co-hexafluorpropylen), Celluloseacetat und Polypropylen umfasst.

13. Therapeutisches System (60, 70) umfassend:
die implantierbare Vorrichtung (100, 600, 700) nach einem der Ansprüche 1-12, wobei die implantierbare Vorrichtung dafür gestaltet ist, subkutan entlang eines Hautbereichs (63, 73) implantiert zu werden; und
eine Zusatzvorrichtung (66, 77, 660), gestaltet zum Zusammenwirken mit der implantierbaren Vorrichtung, um die Zufuhr von Sauerstoff zu der Vielzahl von Zellen zu fördern.

14. Therapeutisches System (60, 70) nach Anspruch 13, wobei die Zusatzvorrichtung (66, 77, 660) umfasst:
(i) ein Transdermalpflaster (77), gestaltet zum Anbringen an eine äußere Hautoberfläche des Hautbereichs (73), wobei das Transdermalpflaster eine Vielzahl von Nadeln (79) umfasst, die einen Stoff (74) tragen, der fähig ist, zu reagieren, um Sauerstoff (75) für die Vielzahl von Zellen (103) in dem Innenbereich (104, 704) des Behälters zu erzeugen, oder
(ii) eine Manschette (66), gestaltet zum Anbringen an der äußeren Hautoberfläche des Hautbereichs (63) und Ausüben von Druck darauf, um einen Blutfluss (64) in Richtung zu der implantierbaren Vorrichtung (100, 600, 700) zu lenken, oder
(iii) ein gesintertes Netz (650), das die implantierbare Vorrichtung umgibt und einen Stoff (630) trägt, der fähig ist, zu reagieren, um Sauerstoff zu erzeugen.

## Revendications

1. Dispositif implantable (100, 700) pour l'administration d'un agent thérapeutique (101), le dispositif implantable comprenant :
un récipient (102, 702) configuré pour contenir une pluralité de cellules (103) capables de produire l'agent thérapeutique au sein d'une zone intérieure (104, 704) du récipient, le récipient délimitant :
des premiers pores (110, 710) délimités par une partie paroi intérieure (107, 707) du récipient, les premiers pores ayant une première taille moyenne qui (i) permet le passage de l'agent thérapeutique et d'oxygène (111) à travers les premiers pores et (ii) empêche le passage de cellules immunitaires (113) à travers les premiers pores, et
des deuxièmes pores (114, 714) délimités par une partie paroi extérieure (108, 708) du récipient, les deuxièmes pores ayant une deuxième taille moyenne qui est plus grande que la première taille moyenne et les deuxièmes pores étant dimensionnés pour favoriser la vascularisation (115) et le transfert d'oxygène le long de la partie paroi extérieure,
dans lequel le récipient a une forme généralement tubulaire, dans lequel la zone intérieure du récipient a une largeur qui est limitée à la réception d'une seule cellule (103) de la pluralité de cellules le long d'un axe central (109, 709) du récipient, de telle sorte qu'au moins une partie d'une surface de chaque cellule de la pluralité de cellules (103) est exposée directement aux premiers pores de la partie paroi intérieure, de telle sorte que la pluralité de cellules peut être agencée au sein du récipient pour recevoir une quantité suffisante d'oxygène pour la survie de la pluralité de cellules à partir de la diffusion passive de l'oxygène à travers les premiers et deuxièmes pores sans une source d'énergie additionnelle pour l'apport d'oxygène supplémentaire à la pluralité de cellules, et
dans lequel le récipient comprend un tube et dans lequel le tube a une configuration en spirale (20) ou une configuration hélicoïdale (30).

2. Dispositif implantable (100, 700) selon la revendication 1, le dispositif implantable ayant un rapport de la surface externe au volume d'environ 200 à environ 5 000.

3. Dispositif implantable (100, 700) selon l'une quelconque des revendications 1 et 2, dans lequel la deuxième taille moyenne des deuxièmes pores est configurée pour permettre le passage de l'agent thérapeutique (101) à travers la partie paroi extérieure (108, 708) vers le système vasculaire adjacent au dispositif implantable.

4. Dispositif implantable (100, 700) selon l'une quelconque des revendications précédentes, dans lequel les premiers pores (110, 710) ont des premières largeurs dans une plage d'environ 10 nm à environ 400 nm et dans lequel les deuxièmes pores (114, 714) ont des secondes largeurs dans une plage d'environ 2 µm à environ 60 µm.

5. Dispositif implantable (100, 700) selon l'une quelconque des revendications précédentes,
dans lequel le récipient (102, 702) a une épaisseur de paroi dans une plage de 1 µm à 100 µm ; et
dans lequel la zone intérieure (104, 704) a une largeur dans une plage d'environ 100 µm à environ 2 mm.

6. Dispositif implantable (100, 700) selon l'une quelconque des revendications précédentes, dans lequel la zone intérieure (104, 704) a une capacité volumique d'environ 0,1 ml à environ 1,5 ml ou une capacité volumique d'environ 0,1 ml à environ 1,1 ml.

7. Dispositif implantable (100, 700) selon l'une quelconque des revendications précédentes, dans lequel la partie paroi extérieure (108, 708) est revêtue d'un facteur de croissance (117) qui favorise la vascularisation (115) et :
dans lequel le facteur de croissance est lié de manière covalente à la partie paroi extérieure et/ou
dans lequel le facteur de croissance est lié électrostatiquement à la partie paroi extérieure et/ou
dans lequel le facteur de croissance est lié de manière spécifique au site à la partie paroi extérieure et/ou
dans lequel le facteur de croissance est choisi dans le groupe constitué par : le facteur de croissance de l'endothélium vasculaire (VEGF), le facteur de croissance placentaire (PDGF), le facteur de croissance transformant bêta (TGF-β) et le facteur de croissance des fibroblastes (FGF) et/ou
dans lequel le facteur de croissance est le facteur de croissance de l'endothélium vasculaire (VEGF), le facteur de croissance placentaire (PDGF),
le facteur de croissance transformant bêta (TGF-β), le facteur de croissance des fibroblastes (FGF) ou une combinaison de ceux-ci et/ou
dans lequel le facteur de croissance est le facteur de croissance vasculaire (VEGF).

8. Dispositif implantable (100, 700) selon l'une quelconque des revendications précédentes, comprenant en outre la pluralité de cellules (103) contenues au sein de la zone intérieure (104, 704) du récipient (102, 702).

9. Dispositif implantable (100, 700) selon la revendication 8, dans lequel la pluralité de cellules (103) sont des cellules bêta et dans lequel l'agent thérapeutique (101) comprend de l'insuline.

10. Dispositif implantable (100, 700) selon l'une quelconque des revendications précédentes, comprenant en outre un ou plusieurs récipients additionnels (40, 50) associés au récipient (102, 702), dans lequel chacun du ou des récipients additionnels contient une pluralité additionnelle de cellules (103).

11. Dispositif implantable (700) selon l'une quelconque des revendications précédentes, dans lequel le récipient comprend une paroi tubulaire (702) qui comprend les parties paroi intérieure (107, 707) et extérieure (108, 708), dans lequel la paroi tubulaire délimite en outre des troisièmes pores (773) agencés radialement entre les premiers et deuxièmes pores, dans lequel les troisièmes pores ont une troisième taille moyenne qui est supérieure à la première taille moyenne et inférieure à la deuxième taille moyenne, dans lequel la paroi tubulaire délimite des pores (770) formés par les premiers, troisièmes et deuxièmes pores de telle sorte que les pores (770) augmentent progressivement en taille le long d'une direction radialement vers l'extérieur (772) depuis une surface intérieure (719) de la paroi tubulaire vers une surface extérieure (716) de la paroi tubulaire et dans lequel le récipient comprend un ou plusieurs matériaux parmi une polycaprolactone, un PTFE, un ePTFE, un nylon, une polyéthercétone, une polyéthersulfone, un polyester, un poly(difluorure de vinylidène) et un polysiloxane.

12. Dispositif implantable selon l'une quelconque des revendications 1 à 10, dans lequel la partie paroi intérieure comprend un élément tubulaire (107) et la partie paroi extérieure comprend un revêtement (108, 173) qui entoure l'élément tubulaire, dans lequel l'élément tubulaire a une première formulation de matériau et le revêtement a une seconde formulation de matériau qui est différente de la première formulation de matériau, dans lequel la première formulation de matériau comprend un ou plusieurs matériaux parmi un polytétrafluoroéthylène expansé (ePTFE), un ester mixte de cellulose, une polyéthersulfone (PES), une PES modifiée, un alginate, un polyéthylèneglycol (PEG), une polyvinylpyrrolidone, un poly(méthylène-co-guanidine), un poly(alcool vinylique), un copolymère de vinylpyrrolidone, l'hydroxypropylméthacylamide, le méthacrylate d'hydroxypropyle, le méthacrylate d'hydroxyéthyle, des poly(oxazolines), l'acide hyaluronique, une polyoxazoline, un polyhydroxypropylméthacrylamide, des polymères zwitterioniques et des polymères contenant des groupes carboxybétaïne, sulfobétaïne et phosphorylcholine et dans lequel la seconde formulation de matériau comprend un ou plusieurs matériaux parmi un poly(difluorure de vinylidène) (PVDF), une polycaprolactone (PCL), un nylon (par exemple un nylon-6), un polytétrafluoroéthylène (PTFE), un ePTFE, une polyéthercétone, une polyéthersulfone, un polyester, un polysiloxane, une polyéthercétone, un poly(fluorure de vinylidène-co-hexafluoropropylène), l'acétate de cellulose et un polypropylène.

13. Système thérapeutique (60, 70) comprenant :
le dispositif implantable (100, 600, 700) selon l'une quelconque des revendications 1 à 12, le dispositif implantable étant configuré pour être implanté par voie sous-cutanée le long d'une zone de la peau (63, 73) ; et
un dispositif accessoire (66, 77, 660) configuré pour coopérer avec le dispositif implantable afin de favoriser l'apport d'oxygène à la pluralité de cellules.

14. Système thérapeutique (60, 70) selon la revendication 13, dans lequel le dispositif accessoire (66, 77, 660) comprend :
(i) un timbre transdermique (77) qui est configuré pour être fixé à une surface extérieure de peau de la zone de peau (73), le timbre transdermique comprenant une pluralité d'aiguilles (79) qui portent une substance (74) susceptible de réagir pour générer de l'oxygène (75) pour la pluralité de cellules (103) au sein de la zone intérieure (104, 704) du récipient, ou
(ii) un brassard (66) qui est configuré pour être fixé à la surface extérieure de peau de la zone de peau (63) et appliquer une pression sur celle-ci pour diriger un flux de sang (64) vers le dispositif implantable (100, 600, 700) ou
(iii) un treillis fritté (650) qui entoure le dispositif implantable et qui porte une substance (630) capable de réagir pour générer de l'oxygène.
